# EUROPEAN PATENT SPECIFICATION

(11) **EP 0 140 556 B1**
(45) Date of publication and mention of the grant of the patent: **15.07.1992**
(21) Application number: 84306233.2
(22) Date of filing: 12.09.1984
(51) Int. Cl.: C12N 15/04, C12N 15/54, C12N 5/00, A01H 1/00

(54) **A T-DNA derived vector**
Von T-DNS abgeleitete vector
Vecteur dérivé du T-ADN

(30) Priority: 14.09.1983 US 532280
(43) Date of publication of application: 08.05.1985
(73) Proprietor: LUBRIZOL GENETICS INC., Wickliffe Ohio 44092 (US)
(72) Inventor: Dahl, Gary A., Madison Wisconsin 53705 (US); Sutton, Dennis W., McFarland Wisconsin 53558 (US); Barker, Richard F., Madison Wisconsin 53716 (US)
(74) Representative: Fisher, Adrian John

(56) References cited:
- WO-A-84/02919
- MOLECULAR & GENERAL GENETICS, vol. 183, no. 2, 1981, Heidelberg, M.DE BEUCKELEER et al. "Further Insight on the Transferred-DNA of Octopine Crown Gall" pages 283-288
- JOURNAL OF MOLECULAR AND APPLIED GENETICS, vol. 1, no. 1, 1981, New York J. LEEMANS et al. "Site-specific Mutagenesis of Agrobacterium Ti Plasmids and Transfer of Genes to Plant Cells" pages 149-164
- PLANT MOLECULAR BIOLOGY, vol. 1, 1982, The Hague, Boston, London G.M.S. VAN SLOGTEREN et al. "The lysopinedehydrogenase gene used as a marker for the selection of octopine crown gall cells" pages 133-142
- MOLECULAR & GENERAL GENETICS, vol. 177, no. 4, 1980, Heidelberg D.J. MERLO et al. "The Boundaries and Copy Numbers of Ti Plasmid T-DNA Vary in Crown Gall Tumors" pages 637-643

## Description

### Field of the Invention

The field of this invention involves the insertion of foreign DNA which is desired to be expressed in a plant, into a transformation vector, i.e., a plasmid capable of introducing the DNA into plant cells and then maintaining that DNA. Plant cells containing foreign genes which have been introduce by this transformation vector, are used to regenerate morphologically normal plants that carry foreign genes. In an ideal situation, these foreign genes should be carried through meiosis to subsequent generations of plants.

A goal of plant genetic manipulation is to introduce desired genes into a plant in such a manner that these genes will be functional in the desired tissue at the correct time. The most promising vehicle for such plant genetic manipulations makes use of the Ti-plasmid of Agrobacterium tumefaciens and the Ri-plasmid of A. rhizogenes. A number of investigators have used the soil organism A. rhizogenes which causes hairy root disease (Costantino, P. et al. (1980) Gene 11:79-87). The transformed plant tissue contains plasmid-derived DNA sequences and the tissue synthesizes an opine resembling agropine (Chilton, M-D., et al. (1982) Nature, London 295:432-434; White, F. F. et al. (1982) Proc. Nat. Acad. Sci. U.S.A. 79:3193-3197). One advantage of A. rhizogenes is that, unlike crown gall tumors, transformed tissue quite easily regenerates into plantlets containing high concentrations of opines.

In addition, specific foreign DNA fragments have been inserted into the T-DNA region of the Ti-plasmids of A. tumefaciens (Leemans, J. et al. (1981) J. Mol. Appl. Genet. 1:149-164; Ooms, G. et al. (1982) Plasmid 7:15-29). Standard in vitro recombinant DNA technology was used to insert a chosen restriction fragment into a specific restriction site lying in a cloned portion of the T-region. After the resulting plasmid was introduced into an A. tumefaciens strain carrying a wild type Ti plasmid, then homologous recombination between the two plasmids produced a Ti-plasmid carrying foreign DNA in the T-region. Tumors produced by infection of plants with A. tumefaciens containing this recombined Ti-plasmid contained the foreign DNA (Garfinkel, D. J. et al. (1981) Cell 27:143-153; Ooms, G. et al. (1981) Gene 14:33-50; Hernalsteens, J. P. et al. (1980) Nature, London 287:654-656).

Transformation vectors, e.g., Ti-plasmids, can carry foreign genes and stably introduce them into plant cells by transfer of the T-DNA regions into the plant genome. These vectors should be able to transform single cells or protoplasts. Such a transformation has been achieved by (i) fusion of bacterial spheroplasts with protoplasts (Hasezawa, S. et al. (1981) Mol. Gen. Genet. 182:206) (ii) the transformation of protoplasts with partially regenerated cell walls by intact bacteria (Marton, L. et al. (1979) Nature, London 277:129-131) and (iii) the delivery of intact Ti-plasmids into protoplasts either as free DNA in the presence of polyethylene glycol and calcium (Krens, F. A. et al. (1982) Nature, London 296:72-74), or encapsulated in liposomes (Draper, J. et al (1982) Plant Cell Physiol. 23:255).

When any of these methods are used, a selectable marker should be available. One possibility is to use antibiotic resistance markers but it would be undesirable to spread such resistance genes in the commercial applications of genetic engineering. Transformed cells which form tumors are generally to be avoided because it is sometimes difficult to regenerate plantlets from such tumor tissue. A possibility of avoiding tumor formation is to use crippled Ti-plasmids which do not cause the formation of tumors but still insert their T-DNA into the plant genome (Leemans, J. et al. (1982) EMBO. J. 1:147-152).

In summary, the field of the present invention is the construction of plant transformation vectors which possess maximum efficiency in the transfer of foreign genes to a plant genome and which then can be amplified in the plant genome whenever desired. These plant transformation vectors should confer maximum expression of these foreign genes in the desired tissues and at the desired time.

### Background of the Invention

Crown gall disease of dicotyledonous plants results from an infection by the gram-negative soil bacterium Agrobacterium tumefaciens. The ability of a strain of A. tumefaciens to transform plant cells and to induce tumors can be correlated to the presence of a large single copy plasmid (the Ti plasmid, which ranges in size from 140 to 235 kilobases). The transformation of plant cells is the result of transferring genetic information, i.e., T-DNA, from the Ti plasmid to the nucleus of the plant cell. Once this transfer has been achieved, the bacterial cell is no longer needed to maintain the transformation (Chilton, M-D., Drummond, M. H., Merlo, D. J., Sciaky, D., Montoya, A. L., Gordon, M. P. and E. W. Nester (1977) Cell 11:263-271).

The transferred T-DNA produces observable phenotypes in the host such as opine synthesis. The earliest opines to be identified resulted from the condensation of an amino acid and a keto acid (Goldman, A., Tempe, J. and G. Morel (1968) Compt. Rend. Acad. Sci. (Paris) 162:630-631). As noted above, plant tumors can be grown in the absence of the causative bacteria and yet they still synthesize opines. Octopine synthase (also named lysopine dehydrogenase) is a single polypeptide chain of molecular weight 38,000 to 39,000 and catalyses the condensation of pyruvate with arginine, histidine, lysine or ornithine (Hack, E. and J. D. Kemp (1980) Plant Physiol. 65:949-955) using NADH or NADPH as co-factor. A second group of opines is produced by nopaline synthase which is a tetramer of four identical polypeptide chains, each of which is approximately 40,000 molecular weight. Again NADH or NADPH is used as the co-factor, but the keto acid is α ketoglutaric acid and the amino acid is arginine or ornithine (Kemp, J. D., Sutton, D. W. and E. Hack (1979) Biochemistry 18:3755-3760). Later, it was shown that a particular strain of A. tumefaciens, which induced tumors synthesizing, for example, octopine could use octopine as a sole source of carbon and nitrogen (Montoya, A. L., Chilton, M. D., Gordon, M. P. Sciaky, D. and E. W. Nester (1977) J. Bacteriol. 129:101-107). Another opine has been identified (Firmin, J. L. and G. R. Fenwick (1978) Nature, London 276:842-844). This opine is agropine and is the result of a condensation reaction between an amino acid and a sugar (Coxon, D. T., Davies, A. M. C., Fenwick, G. R., Self, R. Firmin, J. L., Lipkin, D. and N. F. James (1980) Tetrahedron Letters 21:495-498).

The T-DNA (the DNA transferred from the Ti Plasmid to the plant nucleus) varies from one Ti plasmid group to another. The octopine A-type plasmids transfer two pieces of plasmid DNA to plant cells either together or separately (Thomashow, M. F., Nutter, R., Montoya, A. L., Gordon, M. P. and E. W. Nester (1980) Cell 19:729-739). One piece of a ca. 8 x 10⁶ daltons is always found in tumors with a frequency of one copy/cell whereas another piece of ca. 5 x 10⁶ daltons is sometimes absent and sometimes present at a frequency of up to 30 copies/cell. The two pieces are co-linear on the Ti plasmid restriction endonuclease map but are integrated into the host plant nucleus as separate units. In contrast, the nopaline-type plasmids transfer a single larger piece of Ti plasmid DNA (ca. 10 x 10⁶ daltons) and this is maintained at a frequency of 1-20 copies/tumor cell (Holsters, M. et al. Plasmid 3:212-230).

The transferred T-DNA is integrated into the chromosomal DNA of the transformed plant cells (Thomashow, M. F. et al. (1980) supra) and is extensively transcribed into poly-A-containing mRNA (Gurley, W. B., Kemp, J. D., Albert, M. J., Sutton, D. W. and J. Collins (1979) Proc. Nat. Acad. Sci. U.S.A. 76:1273-1277; McPherson, J. C. et al. (1980) Proc. Nat. Acad. Sci. U.S.A. 77:2666-2670). Part of this transcription codes for the opine synthases (Koekman, B. T. et al. (1979) Plasmid 2:347-357).

Tumor cells containing T-DNA can be maintained in culture indefinitely. Further, unlike normal plant cells, tumor cells can be grown on a chemically defined medium which lacks added auxins and cytokinins (plant growth hormones) (A. C. Braun (1958) Proc. Nat. Acad. Sci. U.S.A. 44:344). Mutants of Ti plasmids can be used to define the location of functional genes such as (i) loci in the T-DNA which determine tumor morphology and opine synthesis (ii) loci outside the T-DNA which are required for virulence and (iii) regions which have no apparent effect on tumorigenicity (Holsters, M. et al. (1980) Plasmid 3:212-230; de Greve, H. et al. (1981) Plasmid 6:235-248; Ooms, G. et al. (1982) Plasmid 7:15-29).

The genetic organization of the T-DNA of a number of tumors promoted by strains of A. tumefaciens containing octopine Ti plasmids has been studied (Merlo, D. J. et al. (1980) Mol. Gen. Genet. 177:637-643; De Beuckeleer, M. et al. (1981) Mol. Gen. Genet. 183:283-288; Thomashow, M. F. et al. (1980) Cell 19:729-739). In some tumor lines, the T-DNA occurs as two segments. The left end of the T-DNA is called T_{L} and includes tms (tumor morphology shoot), tmr (tumor morphology root), tml (tumor morphology large) and, sometimes, ocs (octopine synthase) while the right end is called T_{R}. Tumor maintenance requires T_{L} but not T_{R}. Transformed cells induced by wild type octopine Ti plasmids have been selected in two ways (i) the tissue must be able to form tumors and (ii) the tissue must be able to grow in vitro in the absence of phytohormones. A serious problem associated with the use of wild type Ti plasmids is that it is very difficult to regenerate whole plants from the tumor tissue.

In order to regenerate plants from transformed tissue culture, mutants can be obtained in the tms, tmr and tml loci. However, it then becomes difficult to distinguish transformed tissues from untransformed tissue leading to a requirement for some form of selection. One form of selection has been by the insertion of antibiotic resistance genes into the T-DNA (Ursic, D. et al. (1981) Biochem. Biophys. Res. Comms. 101:1031) (Jiminez, A. et al. (1980) Nature (London) 287:869) (Jorgensen, R. A. et al. (1979) Mol. Gen. Genet. 177:65). In one instance resistance to the antibiotic G418, which is toxic to tobacco cells in culture, was incorporated into the T-DNA. In a second instance, the bacterial transposon Tn5, which confers resistance to kanamycin, was incorporated into the T-DNA. A disadvantage to the use of this type of selection to detect transformed plant tissue is that it leads to needless spread of antibiotic resistance genes. Such a spread of antibiotic resistance genes would be of very large significance if such selection were to be used in the agricultural commercial applications of gene transfer via Ti plasmid vectors. Some other method of differentiating transformed from untransformed plant tissue would be highly desirable especially if such selection were to utilize a normal component of Ti plasmids.

In any program involving the genetic alteration of plants, foreign DNA of interest must be inserted into a transformation vector, i.e., a plasmid, which in turn can stably introduce the DNA into plant cells. These plant cells containing the foreign genes introduced by way of the transformation vector can then be used to regenerate morphologically normal plants. In an ideal situation, the altered plants should transmit the foreign genes through their seeds.

The best available transformation vector at present is the T-DNA fragment which is transferred and integrated into the plant genome from the Ti-plasmid of Agrobacterium tumefaciens following infection. However, if wild type A. tumefaciens is used, then the plant develops a crown gall and it becomes difficult to regenerate whole plants from such crown gall tissue. If mutants in the tumor genes (tms, tmr or tml) are used, then regeneration of plants becomes a practical proposition but selection of transformed tissue becomes difficult. Selection can be re-established by inserting an antibiotic resistance gene into the T-DNA but this method is undesirable for reasons already discussed (see above).

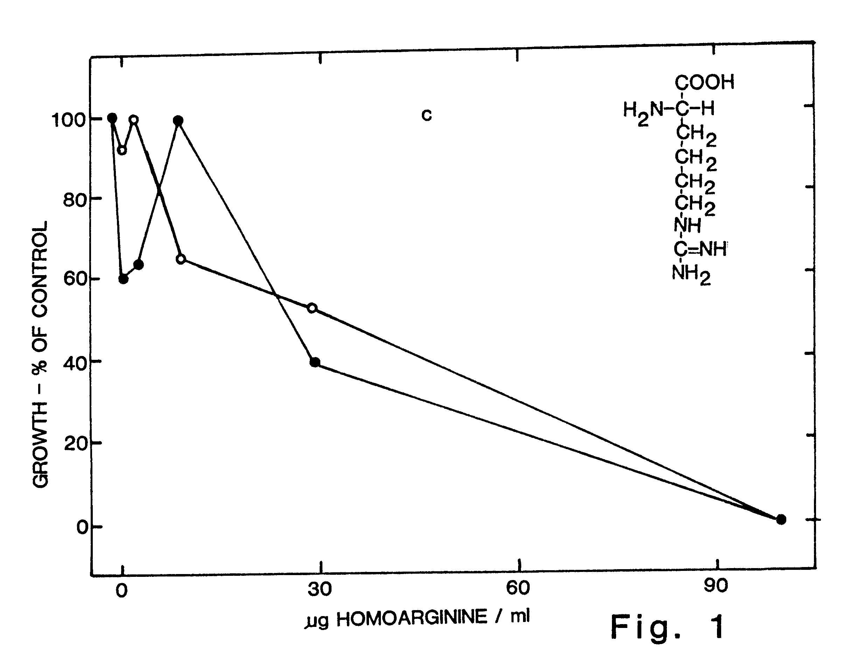

After the work described in the "Detailed Description of the Invention" was completed, Van Slogteren et al. (Van Slogteren, G. M. S. (1982) Plant Mol. Biol. 1:133-142) reported that homoarginine was less toxic to transformed cells than to normal cells. Although homoarginine is a substrate for octopine synthase (Otten, L. A. B. (1979) Ph.D. Thesis, University Leyden, Netherlands; Petit, A. et al. (1966) Compt. Rend. Soc. Biol. (Paris) 160:1806-1807), in independent experiments we did not obtain a selection for transformed cells by use of homoarginine. Little or no selectivity by homoarginine for octopine synthase containing tissues compared to other crown gall or normal tissues was observed (Fig. 1). These independent by us results made it seem unlikely that the use of any amino acid analogs to select transformed tissues and cells would be successful.

### Summary of the Invention

This invention makes use of unique T-DNA constructions from the Ti-plasmid of Agrobacterium tumefaciens to transfer foreign genes for expression in new plants and to recognize transformed cells carrying such foreign genes incorporated into their genomes by selection for an unaltered T-DNA opine synthase gene. Such a selection for transformed cells carrying foreign genes can be done without the development of tumor tissue which makes plant regeneration difficult and without spreading antibiotic resistance genes throughout the plant population.

According to the present invention, there is provided a vector plasmid for the transfer of foreign DNA into the genome of recipient plant cells comprising a heterologous DNA sequence which is expressible in a plant, and which is flanked on each side by a T-DNA repetitive sequence selected from the group consisting of RoTL(A), RoTL(B), RoTR(C) and RoTR(D) having the nucleotide sequences set out hereafter.

Preferably, the vector plasmids of the invention contain a versatile restriction endonuclease site with sticky ends which are compatible to the sticky ends of a number of restriction endonucleases and which can therefore be used for the insertion of foreign DNA fragments obtained with the aid of different restriction endonucleases. In these T-DNA constructions, the tumor forming genes may be deleted and no antibiotic resistance genes need be utilized.

In the presence of a toxic amino acid analog, normal, untransformed cells are unable to grow in culture media or only able to grow extremely slowly, whereas transformed cells containing one or more opine synthase genes are able to detoxify the toxic amino acid analog and so are able to grow. The difference between the growth rates of normal and transformed cells is quite clear when a variety of toxic amino acid analogs are used to select for transformed cells or protoplasts. In the absence of tumor inducing genes, it is easier to regenerate plantlets from tissue culture cells or protoplasts.

Accordingly, the present invention also provides a method of selecting non-tumorous plant cells transformed with a DNA sequence comprising a plant expressible opine synthase gene and a plant expressible heterologous gene, said sequence being flanked on each side by a T-DNA repetitive sequence selected from the group consisting of RoTL(A), RoTL(B), RoTR(C) and RoTR(D) having the nucleotide sequences as defined hereafter from a mixture containing said transformed plant cells and untransformed plant cells comprising:
(a) plating said mixture on a suitable growth medium containing an amino acid analog toxic to normal cells and metabolized by a plant cell expressing the opine synthase encoded by said gene;
(b) growing said mixture on said growth medium for a selected period of time to provide colonies of plant cells; and
(c) selecting from said colonies those colonies exhibiting greater growth rates.

In addition , the T-DNA constructions used in this normal invention permit the recognition of plant cells or protoplasts which have incorporated only parts of TL-DNA, only parts of TR-DNA or parts of both TL-DNA and TR-DNA. Since multiple copies of TR-DNA are often found in a transformed plant genome, this very desirable feature could be used when a high level of expression of foreign genes is required.

### Detailed Description of the Invention

This invention relates to the construction of recombinant plasmids derived from the T-DNA region of Ti plasmids of Agrobacterium tumefaciens. As mentioned above, these recombinant plasmids contain a heterologous DNA sequence which is expressible in a plant, and which is flanked on each side by a T-DNA repetitive sequence selected from the group consisting of RoTL(A), RoTL(B), RoTR(C) and RoTR(D). These repetitive sequences are normally involved in the transfer of T-DNA from the Ti-plasmid to the plant genome. The plasmids may also contain the octopine synthase gene, which normally catalyses the condensation of an amino acid with pyruvate, and/or the agropine/mannopine synthase genes which normally catalyse the condensation of an amino acid with a carbohydrate. The preferred recombinant plasmid constructions described here specifically delete the genes controlling tumor formation and each construction contains a unique BglII site into which foreign DNA fragments encompassing one or more functional prokaryotic or eukaryotic genes can be inserted. Other constructions containing other restriction sites, either substituting for or in addition to the BglII site, may be incorporated, as will be understood by those of ordinary skill in the art.

Plant cells, which have become infected by A. tumefaciens carrying these constructed recombinant plasmids, may have incorporated the T-DNA section. When tumor-formation genes are deleted or rendered inoperative, the cells containing T-DNA do not display the altered morphology and growth habits that normally make transformed cells distinguishable from untransformed cells. In order to recognize which plant cells have incorporated the T-DNA sections of these constructed recombinant plasmids lacking tumor genes into their genomes, the plant cells are grown on certain toxic amino acid analogs disclosed herein. Those cells which carry one or more of certain opine synthase genes can metabolize the toxic amino acid analog to a non-toxic product and will therefore be able to continue growth while those cells which do not contain such opine synthase genes will incorporate the toxic amino acid analog into their proteins resulting in death of such cells.

In order to be able to precisely construct a variety of recombinant T-DNA plasmids with the characteristics described above, the complete nucleotide sequence of T-DNA (22,874 nucleotides) and approximately 900 nucleotides on each side of the T-DNA was obtained. A number of T-DNA restriction fragments were sub-cloned into pBR322 and propagated in either E. coli. strains HB101 or GM33. The individual clones were then sequenced using the method of Maxam and Gilbert [(1977) Proc. Nat. Acad. Sci. U.S.A. 74:560] (see Example 1).

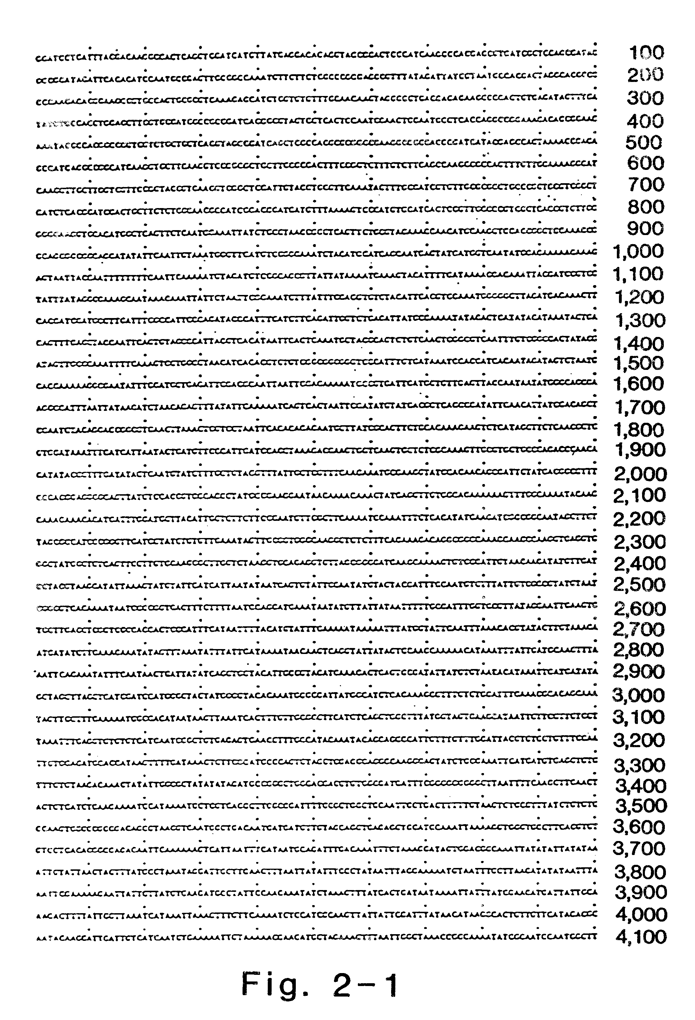

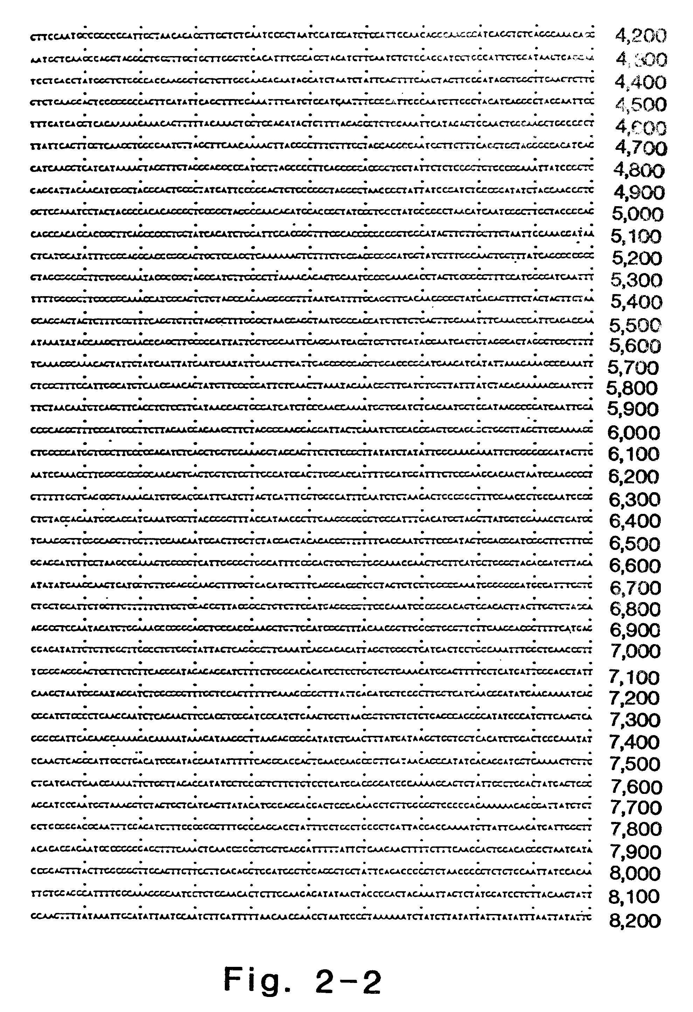

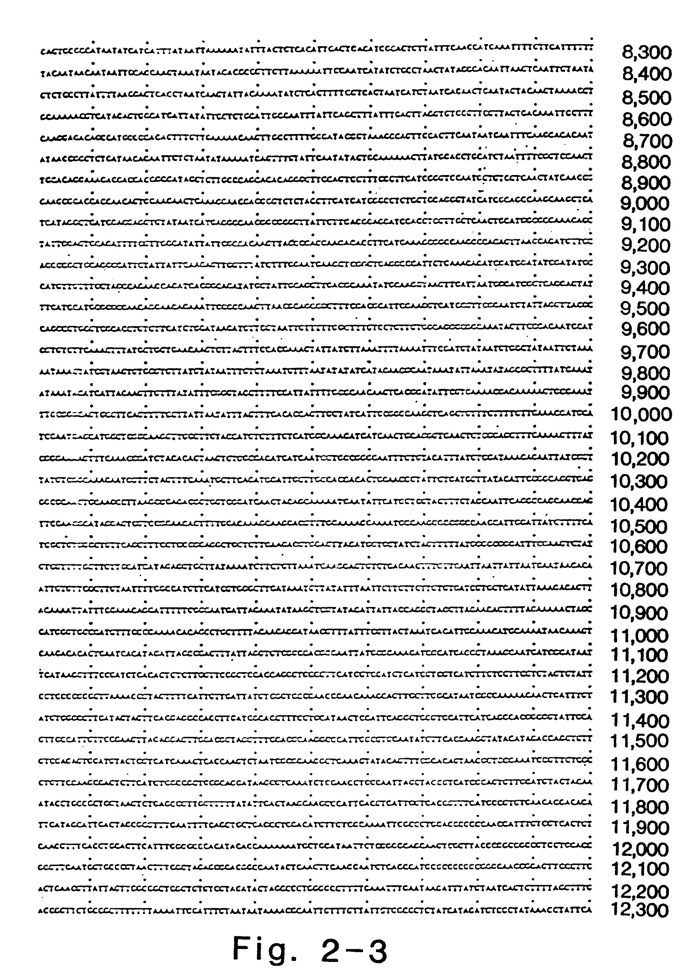

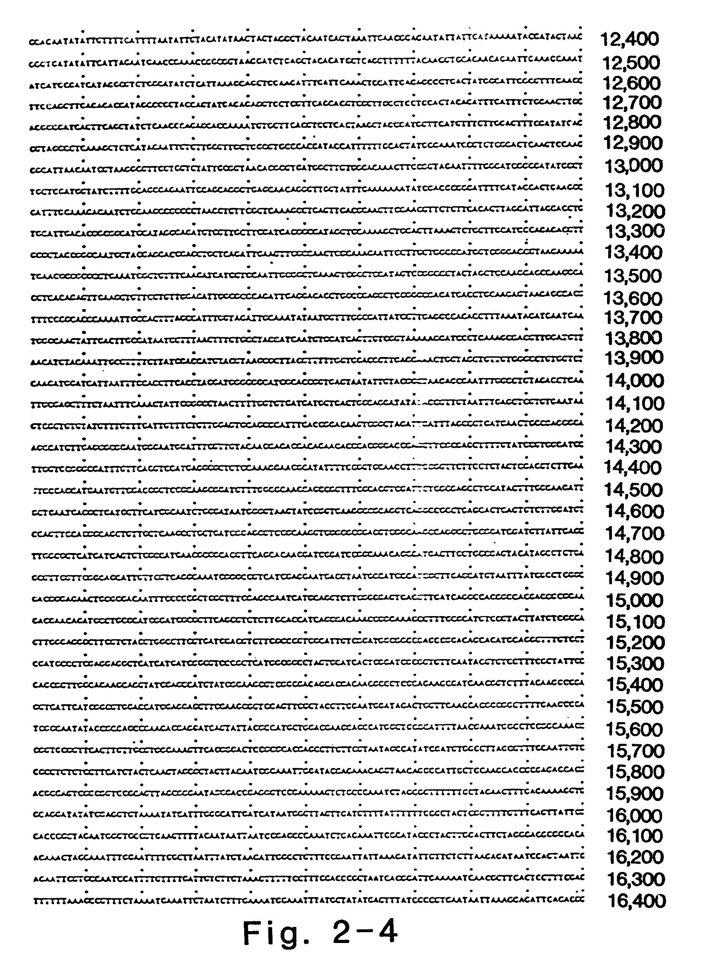

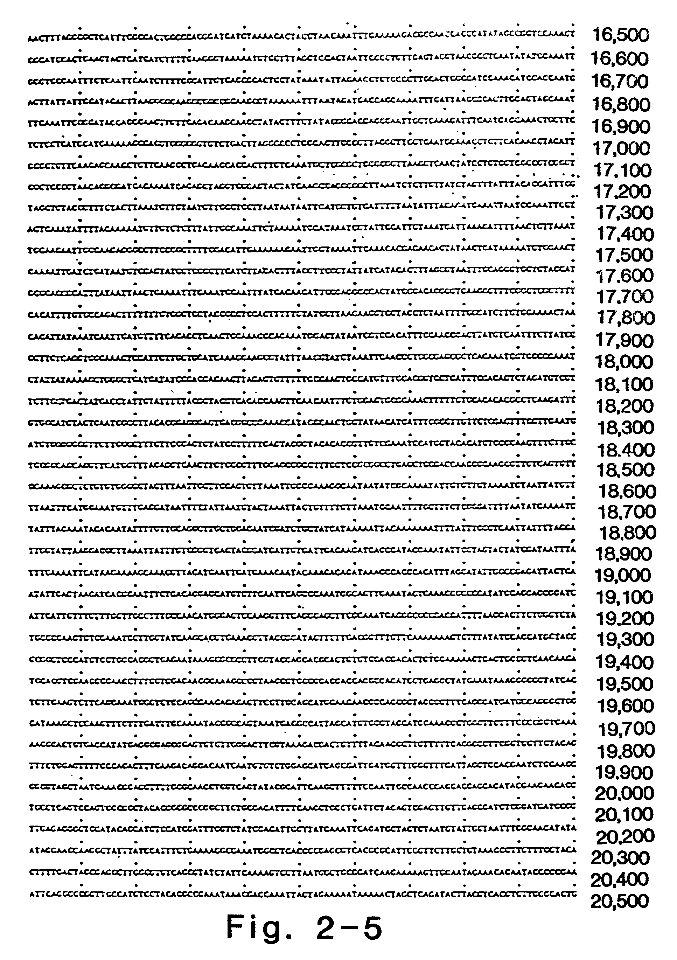

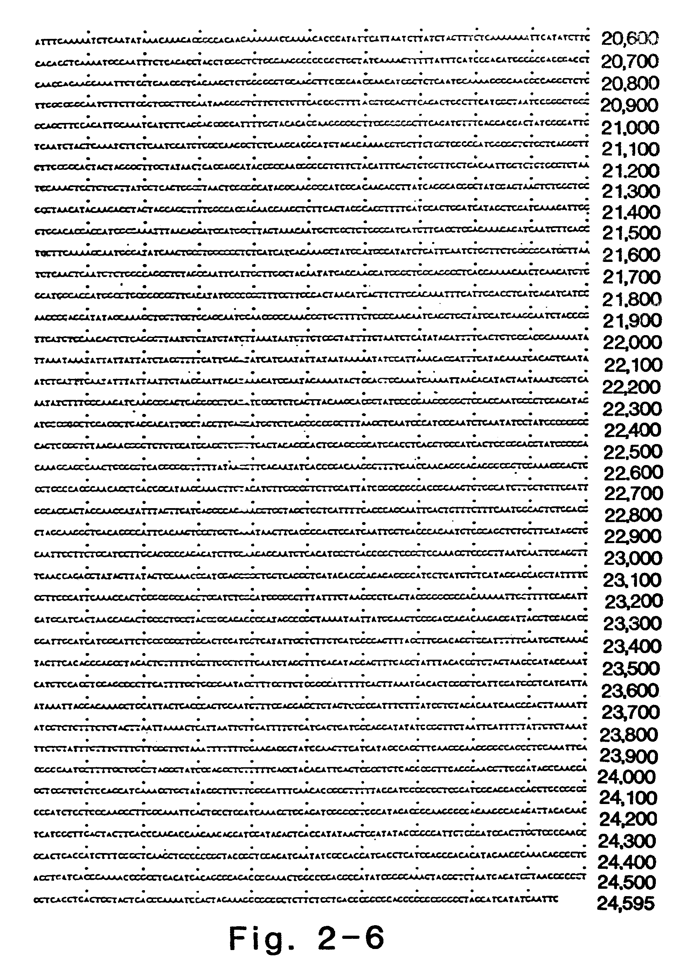

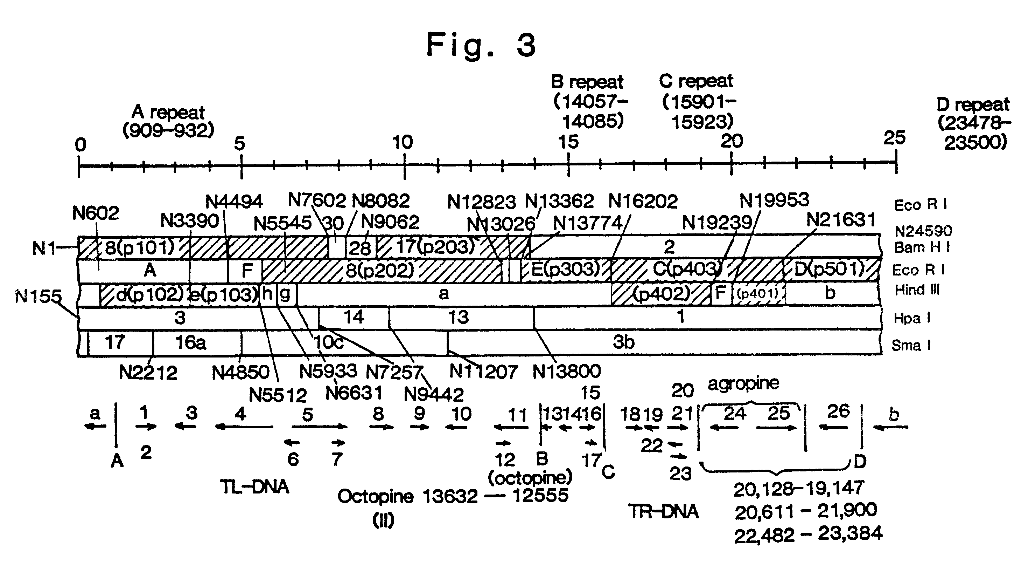

The nucleotide sequence of a portion of the Ti plasmid 15955 containing the T-DNA region is shown (Fig 2). Only one strand of the DNA sequence is presented. It is orientated from 5' to 3' and extends continuously for 24,595 bases, from a BamHI site on the left of fragment Bam8 to an EcoRI site on the right of fragment EcoD (Fig. 3). Both strands were sequenced for 90% of the DNA. The remaining 10% was sequenced on one strand but this was often duplicated by sequencing from different restriction sites.

A restriction endonuclease map of the sequenced region is shown for five different restriction enzymes (Fig. 3). BamHI, EcoRI and HindIII were used to divide the T-DNA region into suitable fragments for subcloning into pBR322. The fragments, indicated by the shaded areas, were used in the construction of T-DNA recombinants cloned into pSUP106 for transfer and subsequent replication in A. tumefaciens. The construction of these T-DNA recombinants was facilitated by a knowledge of the other restriction endonuclease sites (Table 1). Of the 73 enzymes analysed, only sites for EcoK were not present in the Ti sequence. The site locations of enzymes which would digest the DNA more than 30 times are not given.

It has been reported that extended direct repeats of 21-25 bases occur at the borders of the T-DNA (Bevan, M. W., and Chilton, M-D. (1982) Ann. Rev. Genet. 16:357-384). In this work, these two repeats have been shown to start at positions 909 and 23,783, respectively, and they are marked at positions A and D (Fig. 3). These two repeats are referred to hereafter as RoTL(A) and RoTR(D). They are exact direct repeats for 12 bases but they can be extended into 24 base imperfect repeats. Assuming repeats RoTL(A) and RoTR(D) set the outer limits, the total T-region length is 22,874 nucleotides. These border repeat sequences occur in both octopine and nopaline Ti plasmids and play a fundamental role in the transfer of the T-region to the plant genome. In the present study, similar repeated sequences were also found at two locations within the T-region at positions 14,060 (B) and 15,900 (C). These two repeats are referred to hereafter as RoTL(B) and RoTR(C). The presence of these internal repeats is especially interesting because of the observations that the T-DNA in octopine crown gall cells has a complex organization involving one (TL-DNA) or two (TL-DNA and TR-DNA) regions (De Beuckeleer, M. et al. (1981) Mol. Gen. Genet. 183:283-288; Thomashow, M. F. et al. (1980) Cell 19:729-739). The TL-DNA contains the tumor inducing genes (tms, tmr and tml) and occurs in all tumor lines so far examined whereas the TR-DNA occurs only in primary tumors end in some stable tumor lines. The fact that these two T-DNA regions appear to be able to integrate independently and that both are bounded by the basic repeats provides additional evidence of their fundamental role in the transfer of the T-DNA from the Ti-plasmid to the plant genome. The nucleotide sequence of the four repeats is presented here.

Repeat | Nucleotide Sequence

RoTL(A) | G G C A G G A T A T A T T C A A T T G T A A A T

RoTL(B) | G G C A G G A T A T A T A C C G T T G T A A T T

RoTL(C) | G G C A G G A T A T A T C G A G G T G T A A A A

RoTL(D) | G G C A G G A T A T A T G C G G T T G T A A T T

Within the total T-region there are 26 open reading frames (Fig. 3) longer than 300 nucleotides which start with an ATG initiation codon. These possible transcripts would encode polypeptides ranging in size from 11.2 kilodaltons to 83.8 kilodaltons. Fourteen of these open reading frames show possible eukaryotic promoter sequences with close homology to the Goldberg-Hogness box. They also show close agreement to the possible eukaryotic ribosome binding site reported by Kozak (M. Kozak (1978) Cell 15:1109-1123; Kozak, M. [(1979) Nature (London) 280:82-85] and contain possible poly(A) addition sites at their 3'-ends which are thought to act as transcriptional termination signals (Brawerman, G. (1974) Ann. Rev. Biochem. 43:621-642; (1975) Prog. Nucl. Acid Res. Mol. Biol. 17:117-148). Open reading frame 11 codes for octopine synthase while open reading frames 24, 25 and 26 code for mannopine and agropine synthases. It is noteworthy that all the possible eukaryotic transcripts occur within the TL and TR-DNA regions. The open reading frames between the direct repeats RoTL(B) and RoTR(C) and also to the right of RoTR(D) and to the left of RoTL(A) show possible Shine-Dalgarno ribosome binding sites and thus appear to be prokaryotic in origin.

The information made available by the present invention as described above enables the construction of a variety of recombination plasmids eminently useful in the transfer of foreign genes and/or foreign DNA into the genome of recipient plant cells. Foreign genes and/or foreign DNA are here defined as DNA nucleotide sequences which are not normally present in the T-DNA of a Ti-plasmid. Knowledge of the nucleotide sequence provides information on the locations of the promoter regions of the various genes and the boundaries of the open reading frames and therefore, as a corollary, also gives the amino acid sequence and molecular weight of the various encoded proteins. In addition, the nucleotide sequence has located all of the direct repeat regions which are of vital importance in the transfer of the T-DNA (including various foreign genes and/or foreign DNA) from the Ti-plasmid to the plant cell genome. Examples 2 - 7 give details of the construction of a number of useful recombinant T-DNA's which can be inserted into a wide host range plasmid. In each of these constructions, suitably located unique restriction sites are available for the insertion of foreign DNA containing one or more genes. In addition, antibiotic resistance genes are incorporated in the vector region of the recombinant plasmid, i.e., outside the T-DNA repetitive regions which include the selective opine synthase gene and the foreign DNA and/or foreign genes.

A novel feature of these T-DNA recombinant constructions is that the tumor inducing genes (tms, tmr and tml) are preferably deleted or inactivated with the result that it becomes easier to regenerate intact plants from transformed cells. However, it also becomes more difficult to distinguish between transformed and normal cells, since they no longer form tumors and so a new method of selecting transformed cells must be devised. Van Slogteren et al. [(1982) Plant Mol. Biol. 1:133-142] transformed plant cells into octopine-synthesizing tumor cells after infection with a strain of A. tumefaciens carrying an octopine Ti-plasmid.

They claimed that, when small normal shoots and octopine synthase containing crown gall shoots were transferred to solidified agar media containing various levels of homoarginine (HA), growth of normal shoots was clearly inhibited after two to three weeks. In contrast, the crown gall shoots were not inhibited at all at the lower concentrations of homoarginine and only slightly inhibited at the higher concentrations.

Van Slogteren et al. [(1982) supra] also tested the effect of homoarginine on freshly isolated normal and crown gall protoplasts. After three weeks they observed a clear difference (microscopically) in the growth of normal protoplasts compared to crown gall protoplasts. In seven experiments crown gall protoplast showed no growth inhibition and hardly any difference was observed in the crown gall cultures in the presence or absence of HA, whereas in normal cultures in HA the survival rate was estimated to be 10% or less.

Independent experiments by us gave different results. Crown gall tumor lines 15955/1 and 15955/01 were used for these studies: these tissues are derived from single cell clones obtained from tumors incited on N. tabacum cv."Xanthi" by A. tumefaciens strain 15955 (Gelvin, S. B. et al. (1982) Proc. Nat. Acad. Sci. U.S.A. 79:76-80). Both lines grow on medium lacking cytokinins and auxins and both contain T-DNA. However, octopine is not found in line 15955/1, whereas it is found in line 15955/01 reflecting the fact that the octopine synthase gene is not present in the former line, whereas it is present in the latter line. The presence of octopine synthase in line 15955/01 and its absence in line 15955/1 was later confirmed. Thus, these two lines are ideal for testing whether or not the toxic analog homoarginine could be used to select octopine synthase containing genes as claimed by van Slogteren et al. (supra). As can be seen from Figure 1, homoarginine affected the growth of the two tumor lines about equally. Clearly, tissue from a line containing the octopine synthase gene was not distinguishable from a line without the octopine synthase gene. The reason for the discrepancy between our results and those of van Slogteren et al. is at present unclear.

Surprisingly, therefore, further experimentation with a number of other amino acid analogs showed that selection of octopine synthase containing transformed plant cells was successful with some of the analogs but not with others. The relative differences in growth of tissues on medium that contained L-2, 4-diamino-n-butyric acid (an analog of L-ornithine) or 2-thiazolyl-alanine (an analog of histidine) were small and inconsistent from experiment to experiment and work with these analogs was abandoned.

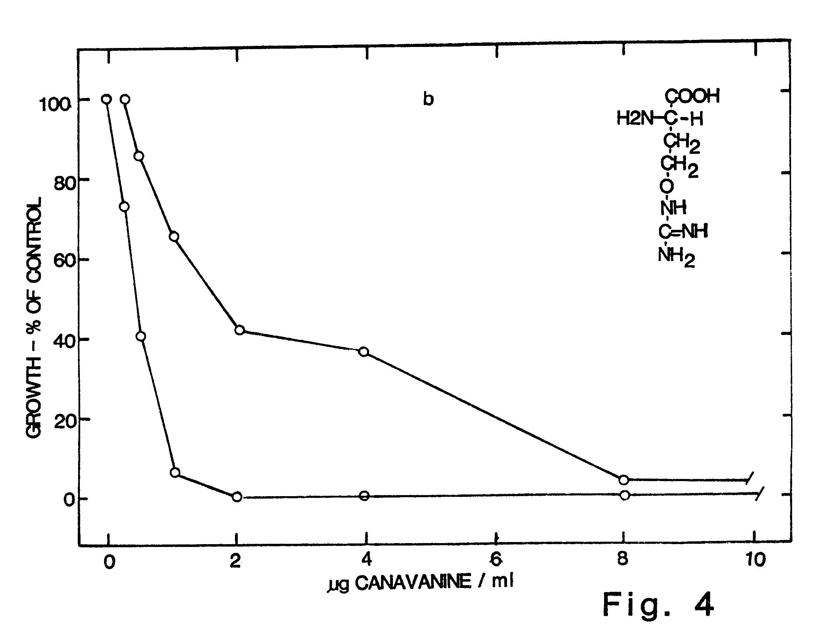

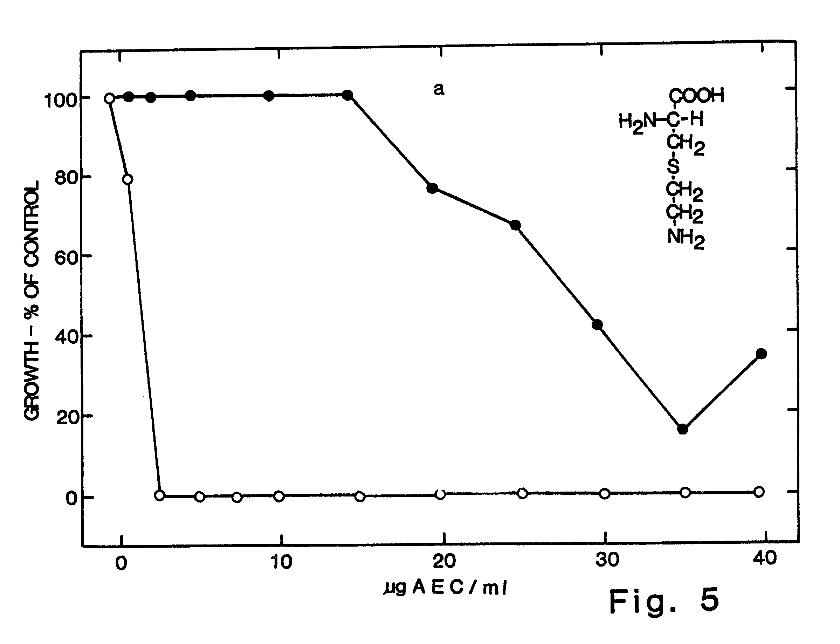

The same two tissue culture lines (i.e., 15955/1 and 15955/01) as were used to test the selective abilities of homoarginine, were also used to test the selective abilities of canavanine-SO₄ (also an analog of arginine) and 2-amino-ethyl-cysteine (an analog of L-lysine). In view of the failure to select for cells with the ability to synthesize octopine synthase by growth on medium containing homoarginine, it was most surprising to find a considerable degree of selection for such cells when tests were conducted on canavanine-SO₄ (CS) (Fig. 4). Even more surprising was the powerful and absolute selection for transformed octopine synthase producing cells when the selection was done on medium containing even very low concentrations of 2-amino-ethyl-cysteine (AEC) (Fig. 5). These two amino acid analogs (CS and AEC) can be used to select for transformed cells in tissue cultures and regenerating plants obtained from these tissue cultures or for transformed protoplasts.

Furthermore, the same approach to selection is useful with the agropine synthase genes. Agropine and mannopine appear to be derived from condensation of glutamine and a carbohydrate and results from this invention have also clearly demonstrated that when agropine/mannopine synthase genes are present in transformed cells, then the use of a toxic amino acid analog distinguishes transformed cells from normal cells. In principle, nopaline synthase could be used to detoxify an appropriate toxic analog of its arginine substrate to achieve selection in the described manner. An appropriate analog has not been identified to date.

A number of strains of Nicotiana tabacum were tested for their ability to grow on toxic analogs of amino acids participating in the agropine-mannopine biosynthetic pathway. Details of the strains used are shown (Table 2).

In all experiments 50mg pieces of tissue were used initially. Three pieces were placed on each petri dish and duplicate dishes were used. These tissue pieces were grown for 9 weeks before they were harvested, dried and weighed. In one set of experiments two strains of Nicotiana tabacum cv. Xanthi were tested. One strain 159 No.-1 was agropine/mannopine negative while the other strain 1590-1 was agropine/mannopine positive. These two strains were tested for their ability to grow on various levels of glutamic-hydrazide (GH-Table 3), S-carbamyl-L-cysteine (CC-Table 5) and 6-diazo-5-oxo-L-norleucine (DON-Table 7). When grown on 20µg GH/ml medium, the growth rate of Strain 159 No.-1 (agropine/mannopine negative) had declined to 9.1% of the control growth rate (Table 3) whereas Strain 1590-1 (agropine/mannopine positive) had only declined to 86.4% of the control rate. The differences between the growth rates of the two strains on this toxic amino acid analog were clear and striking. When these two strains were grown on 25µg CC/ml medium, there was a clear drop in the growth rate of the agropine/mannopine negative strain to 6.9% of the control growth (Table 5) whereas there was essentially no drop in the growth rate of the agropine/mannopine positive strain. Finally, when the agropine/mannopine negative strain was grown on 0.25µg DON/ml medium, there was no growth whatsoever, whereas the agropine/mannopine positive strain had only declined to 24% of the control growth rate (Table 7).

In a second set of experiments, an agropine/mannopine negative strain (WC 58-Table 2) and an agropine/mannopine positive strain (W-B634) of Nicotiana tabacum cv Wisconsin 38 were tested on various levels of GH, CC and DON. In the case of GH (Table 4), the agropine/mannopine negative strain WC58 was dead in the presence of 10µg GH/ml of medium, whereas the growth rate of the agropine/mannopine positive strain W-B634 was still 55% of the control growth rate. When the same two strains were tested on CC (Table 6), the agropine/mannopine negative strain was dead in the presence of 50µg CC/ml of medium while the positive strain showed no decrease at all in the growth rate. Finally, the results were also quite clear cut when these strains were grown on DON (Table 8). In the presence of 0.12µg DON/ml of medium, the agropine/mannopine negative strain (W-C58) was dead while the agropine/mannopine positive strain (W-B634) had grown to some extent (i.e., 12% of the control growth rate).

Thus, the results revealed in this invention teach a unique method of selecting for transformed tissues, cells and protoplasts. These results make use of the fact that untransformed cells lacking the genes for opine synthesis cannot grow in the presence of toxic amino acid analogs, whereas transformed cells containing opine synthases are able to grow. Furthermore, the selection can be used either when the octoplne synthase genes are present or when the agropine/mannoplne synthase genes are present.

The ability to select for transformed cells by selecting for the octopine synthase or the agropine/mannopine synthase genes permits a further refinement in the engineering of foreign genes which are desired to be expressed. Thus, the transformed cells may be obtained by selecting for one of the opine synthase biosynthetic pathways and inserting the foreign gene(s) into the reconstructed T-DNA in such a manner that expression of these genes comes under the control of the other opine synthase. For example, it would be possible to construct a T-DNA containing RoTL(A), octopine synthase, RoTL(B), agropine/mannopine synthase-RoTR(D). An analog of lysine, e.g., 2-amino-ethyl-cysteine could be used to select for transformed cells by selecting for octopine synthase while the unique MstII site (nucleotide 19471 within the agropine/mannopine genes) could be used to insert any foreign DNA.

A further advantage of such a T-DNA construction would be that the two repetitive sequences RoTL(A) and RoTL(B) as well as the octopine synthase gene used to select for transformed gene(s) which would still be expressed under the control of the agropine/mannopine synthase gene promoter region.

In summary, the use of reconstructed T-DNA plasmids containing only the direct repeats involved in incorporation of the T-DNA into the plant genome and one or more opine synthesizing genes has resulted in the following useful results: 1. The tumor inducing genes have been deleted resulting in greater success of plant regeneration from transformed tissue cultures or protoplasts. 2. The opine synthesizing genes have been used to select for those plant cells which have incorporated the T-DNA (and therefore in addition any foreign genes which have been inserted). 3. The invention now permits the recognition of plant cells which have incorporated only parts of TL, only parts of TR or parts of both TL and TR. Since multiple copies of TR are found in a transformed plant genome, this feature could result in a much higher level of expression of foreign genes incorporated into some of these T-DNA constructions.

### Example 1: Sequencing of the nucleotides of the T-DNA from the Ti plasmid pTi 15955

Fragments of T-DNA and the flanking regions obtained by use of restriction endonucleases were cloned into pBR322 and then propagated in either E. coli strains HB101 or GM33. The individual clones were then sequenced using the method of A. M. Maxam and W. Gilbert [(1977) Proc. Nat. Acad. Sci. U.S.A. 74:560]. For sequencing 10µg of the cloned DNA's were cut with a suitable restriction enzyme and then treated for 30 minutes at 55°C with 2.5 units of calf intestinal alkaline phosphatase after the pH was adjusted by adding one-tenth volume of 1.0 M Tris/HCl pH 8.4 to the reaction tube. The alkaline phosphatase was removed by three phenol extractions followed by two ethanol precipitations. The dephosphorylated DNA was then dried and taken up in 15µl water and 15µl denaturation buffer consisting of 20mM Tris/HCl, pH 9.5, 1mM spermidine and 0.1 mM EDTA. This mixture was incubated at 70°C for 5 minutes and then immediately put into iced water. After chilling, 4µl of kinase buffer consisting of 500 mM Tris/HCl pH9.5, 100 mM MgCl₂, 50 mM dithiothreitol, 50% (v/v) glycerol, 100µ Ci of [ -³²p] ATP and 2.0 units of polynucleotide kinase were added and the reaction mixture was incubated at 37°C for 30 minutes. The reaction was stopped by ethanol precipitation and the sample dried. The double end-labelled DNA was digested with a suitable restriction enzyme to produce single end-labelled fragments which were then separated on and eluted from a polyacrlamide gel (procedures 4, 5a, 7 and 9 of Maxam and Gilbert). The DNA sequencing reactions were then carried out as described (Maxam, A. M. and W. Gilbert 1977) supra) with the following modifications. The limiting G + A reaction was carried out by adding 30µl of 88% formic acid to the reaction mix and incubating at 20°C for 3 minutes. The reaction was stopped by the addition of 400µl 0.3M Na-acetate (hydrazine stop). The G reaction time was reduced to 20 seconds and incubated at 20°C. The C + T and C reactions were reduced to three minutes at 20°C and stopped by the addition of 400µl hydrazine stop. All the reactions were then continued as described (Maxam, A. M. and W. Gilbert (1977) supra). Long sequencing gels 20cm wide, 10cm length and 0.2 mm thick were used to separate the oligonucleotides. The gel plates were treated with a silane (Garoff, H. and Ansorge, W. (1981) Anal. Biochem. 115:450-457) to bind the acrylamide chemically to one face plate. The other supporting plate is a thermostating plate which maintains the gel at 50° throughout electrophoresis. Samples were separated on 4%, 6% and 16% acrylamide gels. Differential time loadings were avoided by applying each sample to each of the three gels simultaneously. Gels were run for 14 hours at 3,000 volts to provide adequate cross-over of the sequencing ladders from gel to gel. After electrophoresis, the gel (bonded to the face plate) was fixed in 10% acetic acid for 15 minutes, then rinsed in water, The gel dried directly onto the face plate, shrinking to a thickness of approximately 0,01mm. Consequently, X-ray film could be placed in direct contact with the dried gel resulting in increased band intensity and resolution. Auto-radiography was carried out at room temperature without the use of intensifying screens. Using these techniques, it was possible to routinely sequence 500 base pairs per fragment. Therefore, by applying 5 fragments to each set of 3 gels, 2500 bases of sequence could be obtained. Analysis of the DNA and protein sequences were done by computer. The programs used were those of Dr. 0. Smithers and Dr. F. Blattner (University of Wisconsin, Madison).

### Example 2: Construction of a micro-Ti plasmid including RoTL(A), RoTL(B) and the octopine synthase gene

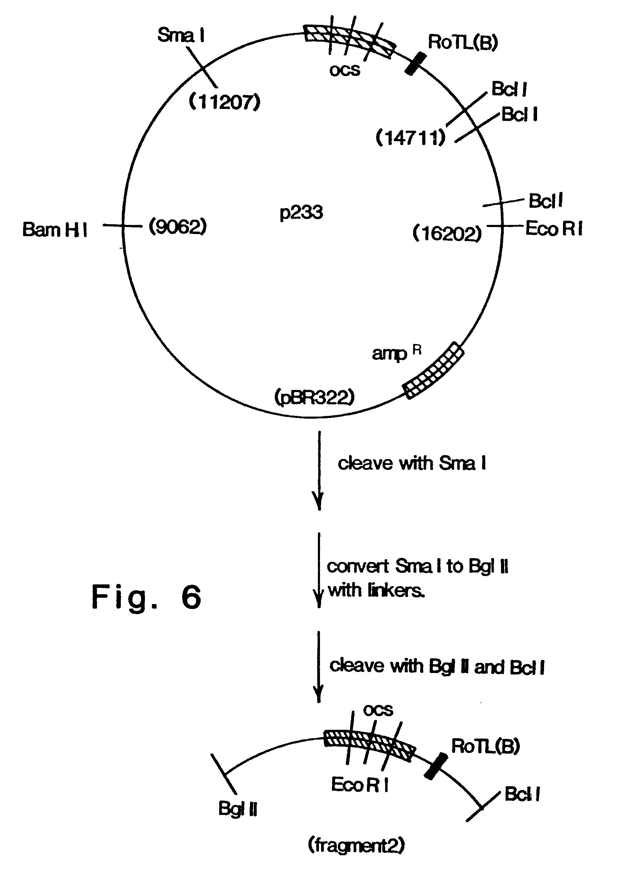

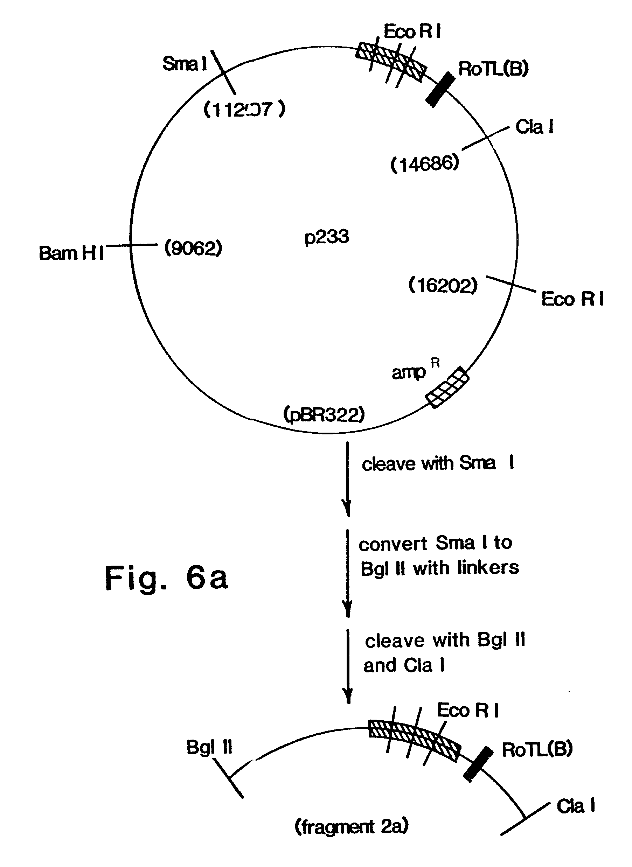

Clone p233 consiste of the BamHI fragment 17 and EcoRI fragment E (Figs. 6 and 6a) cloned into the vector pBR322 (Bethesda Research Laboratories, Inc., P.O. Box 577, Gaithersburg, MD 20760). The clone was cleaved with the restriction endonuclease SmaI (Fig. 6a), the blunt ended SmaI restriction site was converted to a BglII site by the use of BglII linkers and the DNA was ligated and then transformed into E. coli K802. The resulting recombinant plasmid was purified and then transformed into E. coli GM33 which is Dam⁻ and therefore incapable of methylating adenine residues. In this strain the ClaI site at nucleotide 14686 was not methylated and could be cleaved. Following ClaI and BglII digestion, the procedure yielded a T-DNA fragment (fragment 2a from nucleotide 11207 to nucleotide 14686) containing the complete octopine synthase gene and the direct repeat at the right hand border of TL (i.e., RoTL(B)). Alternatively, a similar sized fragment (fragment 2) can be obtained by use of the restriction endonuclease BclI in place of ClaI (Fig. 6).

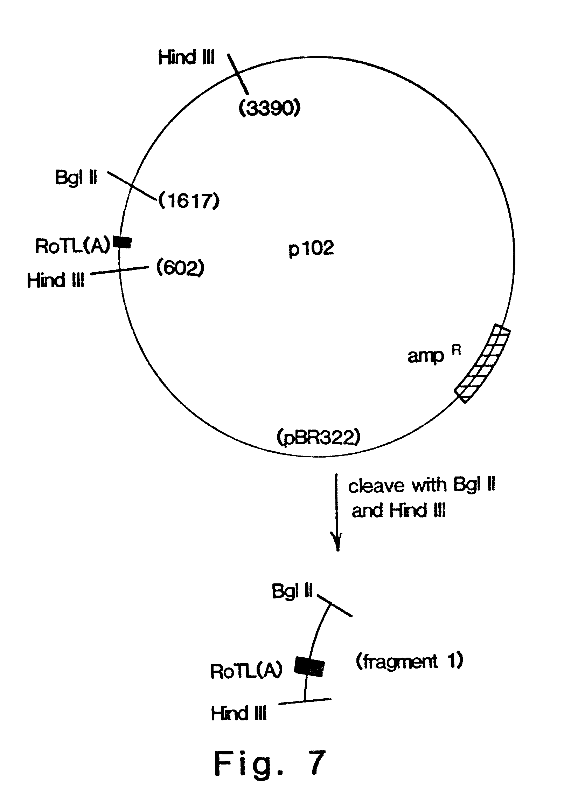

Next the HindIII clone p102 (covering T-DNA nucleotides 602-3390) previously cloned into the vector pBR322 and containing a BglII site (nucleotide 1617) was cleaved with BglII and HindIII (Fig. 7) to yield a T-DNA fragment (fragment 1) containing the direct repeat at the left hand border of T_{L} (i.e., RoTL(A)).

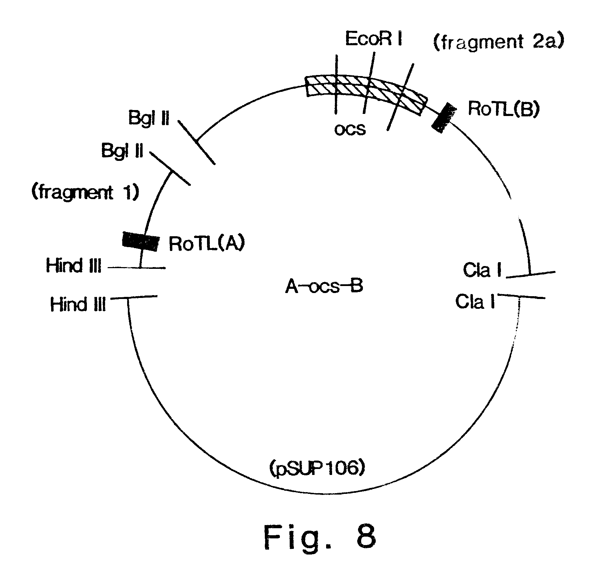

Finally, the wide host range plasmid pSUP106 which will replicate in Agrobacterium tumefaciens was cleaved with HindIII and ClaI (Fig. 8). These three fragments have the following restriction sites at their borders: Fragment 1 has BglII and HindIII; fragment 2 has BglII and ClaI; and pSUP106 has ClaHI and HindIII. Since the restriction sites bounding the three fragments are never common to more than two fragments, ligation can only occur in one arrangement (A-ocs-B) (Fig. 8). The BglII site between fragments 1 and 2 is unique site and can be used to insert any foreign DNA fragment as desired. This restriction site is very versatile since foreign DNA segments which have been cleaved by BglII, BamHI, BclI, MboI and Sau3A, among others, can be inserted.

Restriction enzymes and ligase were all used according to the recommendations of the supplier.

### Example 3: Construction of a micro-Ti plasmid including RoTL(A), octopine synthase, RoTL(B) RoTR(D)

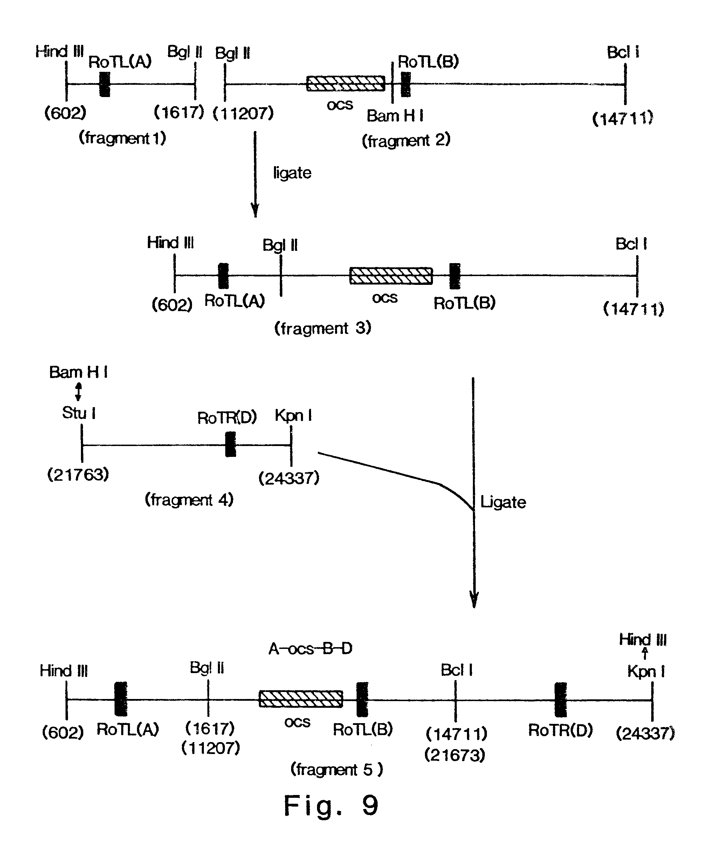

As described in Example 2, the HindIII clone p102 (spanning T-DNA nucleotides 602-3390) was used to yield a T-DNA fragment 1 (Fig. 7) and clone p233 was used to produce fragment 2 (Fig. 6). These two fragments were ligated to produce fragment 3 (Fig. 9) with a HindIII site at one end and a BclI site at the other end. Fragment 3 contains the nucleotide sequences for RoTL(A), octopine synthase and RoTL(B). It should be noted that RoTL(B) could be omitted from the reconstructed T-DNA plasmid by restriction cleavage with BamHi at the BamHI restriction site at nucleotide 13774, i.e., 142 nucleotides upstream from the initiation codon of octopine synthase. When this site was used, it was found that the octopine synthase gene expression was prevented. When the BclI site (nucleotide 14711) was used to isolate a fragment containing the octopine synthase gene, then the gene was actively expressed. It is possible that a different DNA fragment could be substituted for the region between BclI and BamHI to restore expression of the octopine synthase gene, e.g., any of the repetitive sequences, i.e., RoTL(A), RoTL(B), RoTR(C) or RoTR(D).

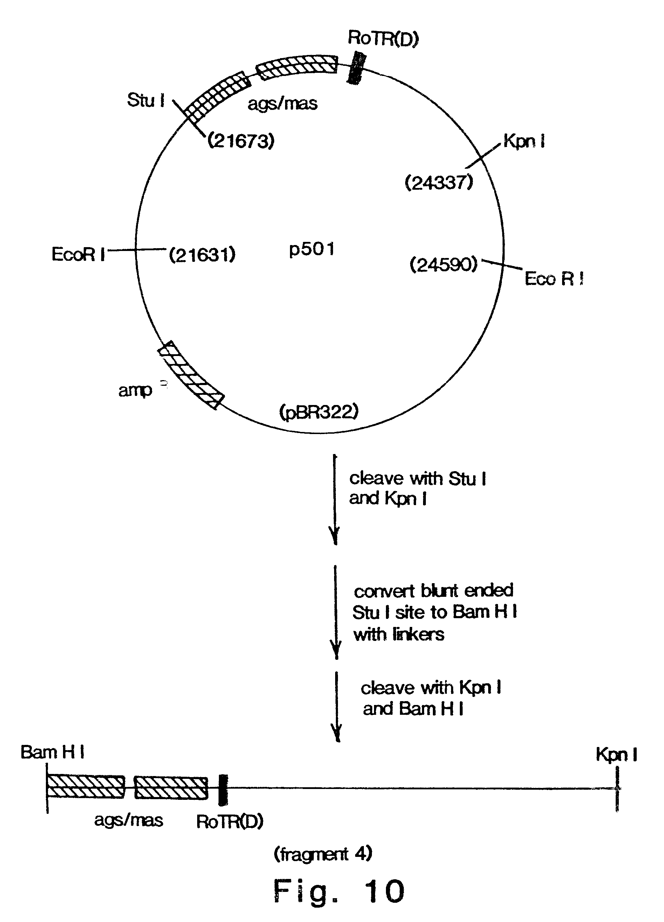

The next step is to amplify the EcoRI clone p501 (Fig. 10) previously cloned into pBR322 and to purify the plasmid. The purified plasmid is then cleaved with restriction endonucleases StuI and KpnI to yield a T-DNA fragment covering nucleotides 21673 to 24337 and including the repetitive sequence RoTR(D) (fragment 4). The cleavage produced by StuI is blunt ended, whereas that produced by KpnI has a 3'-overhang. The blunt-ended StuI site is converted to a BamHI site by the use of BamHI linkers. After BamHI digestion, fragments 3 and 4 are ligated together to yield fragment 5 (Fig. 9) which has a HindIII site at one end and a KpnI site at the other end. It should be noted that the endonucleases BclI and BamHI produce compatible cohesive ends.

Then the 3'-overhang produced by cleavage of fragment 4 with KpnI is made blunt ended using the 3'-exonuclease activity of bacteriophage T4 DNA polymerase (Maniatis, T. et al. (1982) In Molecular Cloning p.140 Cold Spring Harbor). Following inactivation of the polymerase by phenol extraction and precipitation of the plasmid in cold ethanol, the blunt end is converted to a HindIII site by the use of HindIII synthetic linkers so that fragment 5 now has HindIII sites as both ends. Fragment 5 is then digested with the enzyme HindIII.

Finally the wide host range plasmid pSUP106 is linearized with HindIII and treated with bacterial alkaline phosphatase. Fragment 5 is then ligated into the linearized pSUP106 to yield a recombinant plasmid in which the T-DNA section contains in sequence RoTL(A), octopine synthase, RoTL(B), and RoTR(D) (A-ocs-B-D) (Fig. 9). It should be noted that the versatile BglII site at the junction of fragments 1 and 2 can be used to insert any foreign DNA fragment carrying one or more genes that are desired to be expressed.

### Example 4: Construction of a micro-Ti plasmid including RoTL(A), RoTR(D) and the agropine/mannopine synthase

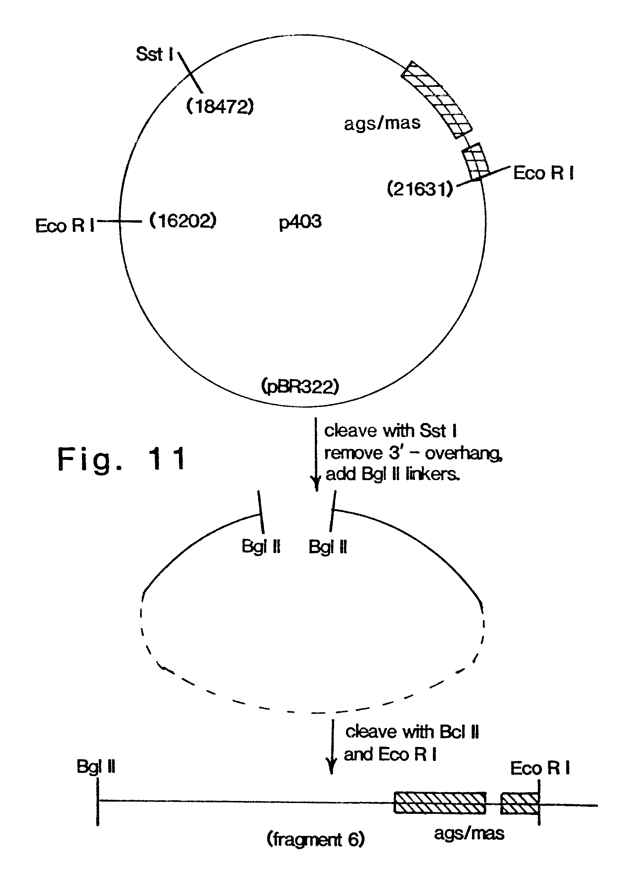

The agropine mannopine synthase genes include open reading frames 24, 25, and 26 (Fig. 3) but it is not yet known which of these open reading frames corresponds to agropine synthase and which corresponds to mannopine synthase. Reading frames 24-26 are therefore referred to as agropine/mannopine synthase genes (ags/mas). The EcoRI clone p403 (T-DNA nucleotides 16202 to 21631) and the EcoRI clone p501 (T-DNA nucleotides 21632 to 24590) were both cloned into pBR322 and separately transformed into E. coli HB101. Following amplification and purification, clone p403 is cleaved with SstI at the T-DNA nucleotide 18,472 (see Fig. 11). The overhanging 3'-end is made blunt ended using the 3'-exonuclease activity of bacteriophage T4 DNA polymerase (Maniatis, T. et al (1982) In Molecular Cloning p.140 Cold Spring Harbor). Following inactivation of the polymerase by phenol extraction and precipitation of the plasmid in cold ethanol, the blunt ends are converted to BglII sites by the use of BglII synthetic linkers. Then the DNA is cleaved with EcoRI and BglII to produce a fragment from the T-DNA covering nucleotides 18,472 to 21,631. This fragment (fragment 6) contains some of the agropine/mannopine synthase genes and is bounded by a BglII site and an EcoRI site.

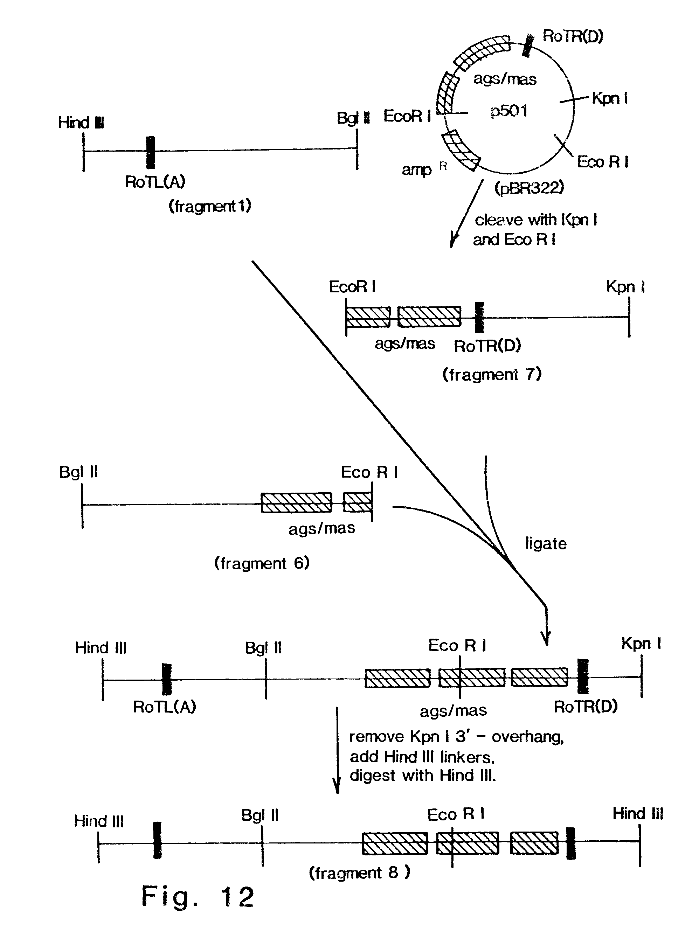

Next EcoRI clone p501 (T-DNA nucleotides 21632-24590), previously cloned into pBR322, is cleaved with restriction endonucleases EcoRI and KpnI. The resulting fragment 7 (Fig. 12) contains the remainder of the agropine/mannopine synthase genes (not included in fragment 6) and, in addition carries the repetitive sequence RoTR(D).

Fragment 1 (Fig. 7), fragment 6 (Fig. 11) and fragment 7 (Fig. 12) are mixed together and ligated. Since no more than two of the six ends have compatible cohesive ends, the three fragments can only ligate in one orientation to give fragment 8 (Fig. 12) (A-ags/mas-D) with a HindIII site at one end and a KpnI site at the other end. Finally, the 3'-overhang at the KpnI site is removed by T4 DNA polymerase and converted to a HindIII site by use of synthetic linkers. (Note: this will convert both ends to blunt ends and add HindIII linkers to the HindIII end. This adds 4bp to that end.) this fragment therefore has HindIII sites at both ends and can be inserted into pSUP106 after this wide host range vector has been linearized by restriction endonuclease HindIII.

### Example 5: Construction of a micro-Ti plasmid including RoTL(B), RoTR(D) and the agropine synthase genes.

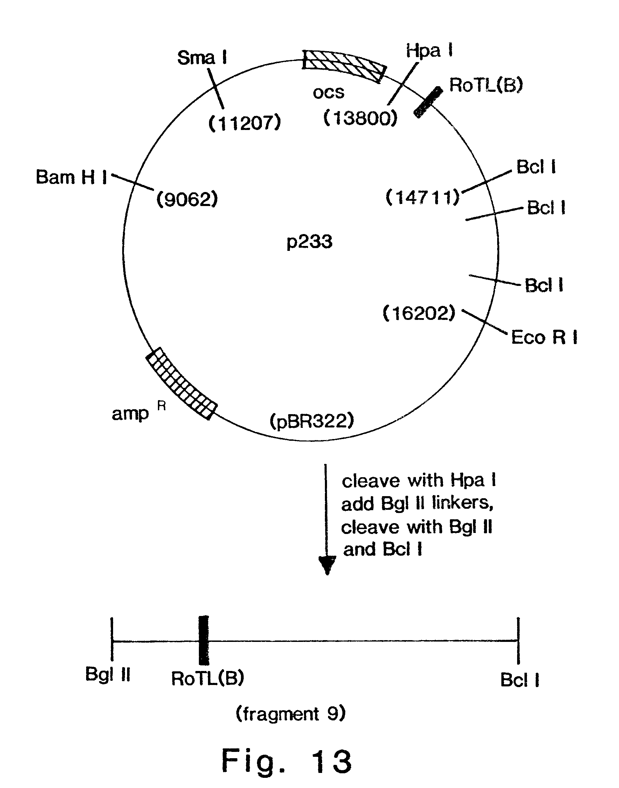

Clone p233 which spans the BamHI fragment 17 and EcoRI fragment E (Fig. 13) was cloned into the vector pBR322. The resulting recombinant plasmid was then transformed into E. coli GM33 which is Dam⁻ and was therefore incapable of methylation. In this strain the BclI site at nucleotide 14711 was not methylated and could be cleaved. Following amplification, the recombinant plasmid is purified and cleaved with the restriction endonuclease HpaI (Fig. 13). The blunt ended HpaI restriction site is converted to a BglII site by the use of BglII linkers. Then the fragment is cleaved with the restriction endonucleases BglII and BclI. This procedure yields a T-DNA fragment (fragment 9 from nucleotide 13,800 to nucleotide 14711) which contains the direct repeat at the right hand border of T_{L} (i.e., RoTL(B)).

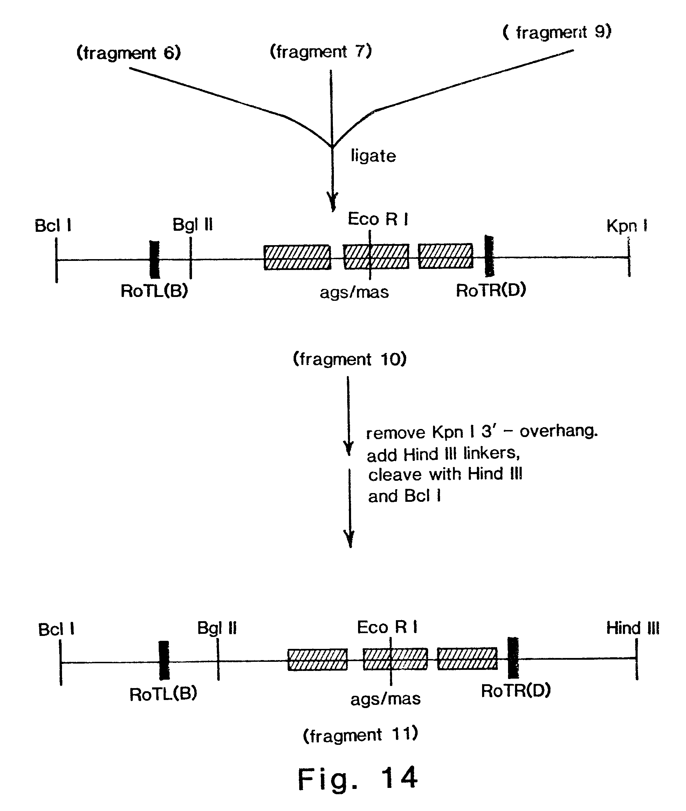

A second fragment (fragment 10) which includes the agropine/mannopine synthase genes and the direct repeat at the right hand border of T_{R} (i.e., RoTR(D)) was constructed by mixing fragments 6 and 7 (Fig. 12) with fragment 9 (Fig. 13) and ligating them together to give fragment 10 (Fig. 14). The 3'-overhang at the KpnI restriction site is then blunt ended by use of T4 DNA polymerase and converted to a HindIII site by use of synthetic linkers. This procedure will convert both ends to blunt ends and add HindIII linkers to the HindIII end. Subsequent digestion with HindIII and BclI will produce a fragment with a BclI site at one end (compatible with a BamHI site) and a HindIII site at the other end (fragment 11-B-ags/mas-D) (Fig. 14).

The wide host range vector pSUP106 which can replicate in Agrobacterium tumefaciens is then linearized by digestion with BamHI and HindIII and fragment 11 is inserted into the linearized vector.

### Example 6: Construction of a micro-Ti plasmid including RoTL(A), the agropine/mannopine synthase genes, RoTR(D), the octopine synthase gene and RoTL(B)

Fragment 1 was obtained as described in Example 2 (Fig. 7). This fragment has a HindIII site at one end and a BglII site at the other end. Fragment 12 was obtained by ligation of fragments 6 and 7 (Fig. 12) and has a BglII site at one end and a KpnI site at the other end.

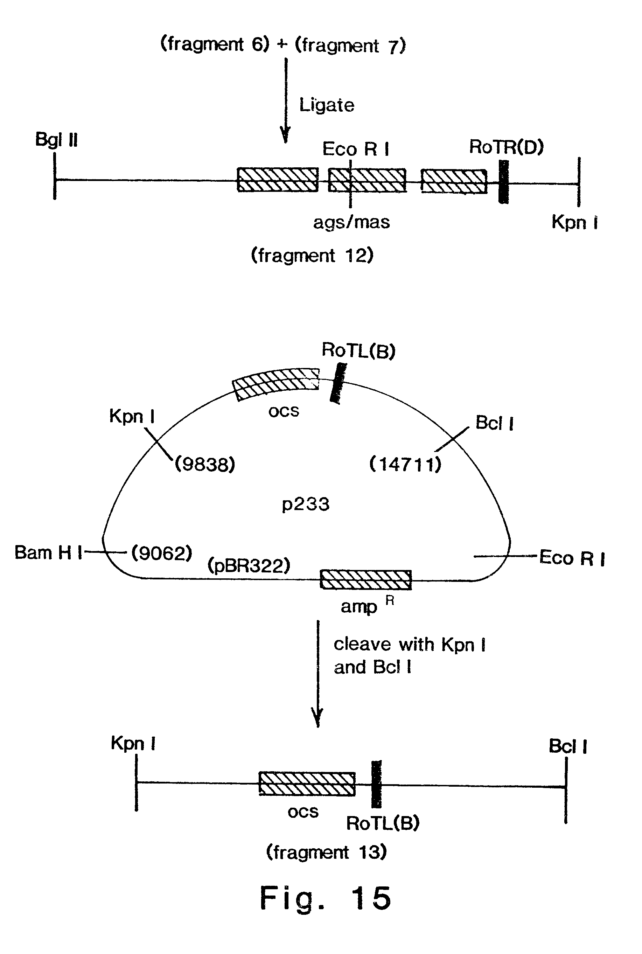

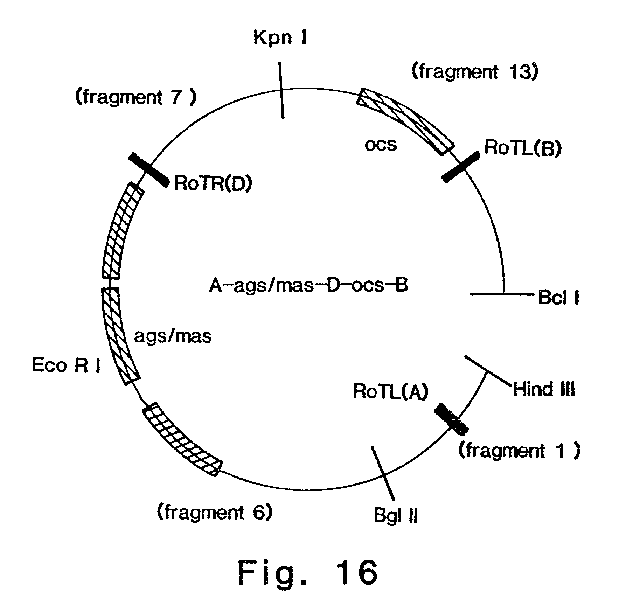

Clone p233 which spans the BamHI fragment 17 and EcoRI fragment E (Figs. 6 and 13) was cloned into the vector pBR322. The resulting recombinant plasmid was then transformed into E. coli GM33 which is Dam⁻ and was therefore incapable of methylation. In this strain the BclI site at nucleotide 14711 was not methylated and could be cleaved with the enzyme BclI. Following amplification, the recombinant plasmid is purified and cleaved with the restriction endonucleases BclI and KpnI to yield fragment 13 spanning T-DNA nucleotides from the KpnI site (nucleotide 9838) to the BclI site (nucleotide 14711). Ligation of a mixture of fragment 1 (Fig. 7), fragment 12 (Fig. 12) and fragment 13 (Fig. 15) yields a recombinant fragment 14 (Fig. 16) with a HindIII site at one end and a BclI site at the other end (A-ags/mas-D-ocs-B).

The wide host range plasmid pSUP106 is finally linearized with the restriction endonucleases BamHi and HindIII. After linearization, fragment 14 is inserted by ligation.

This construction has the advantage that, if desired, the foreign genes can be inserted into one of the opine synthase genes and the promoter of that gene then used for the expression of the foreign genes while the second opine synthase gene can be used for the selection of transformed cells.

### Example 7: Construction of a micro-Ti plasmid including RoTL(A), duplicate octopine synthase genes and RoTL(B) and RoTR(D)

The methods of obtaining the fragments used in the present T-DNA construction have been described in the previous examples. Fragments 1 and 2 were obtained as described in Example 2 (Figs. 6 and 7). Fragment 4 is obtained as described in Example 3 (Fig. 10) and fragment 13 is obtained as described in Example 6 (Fig. 15).

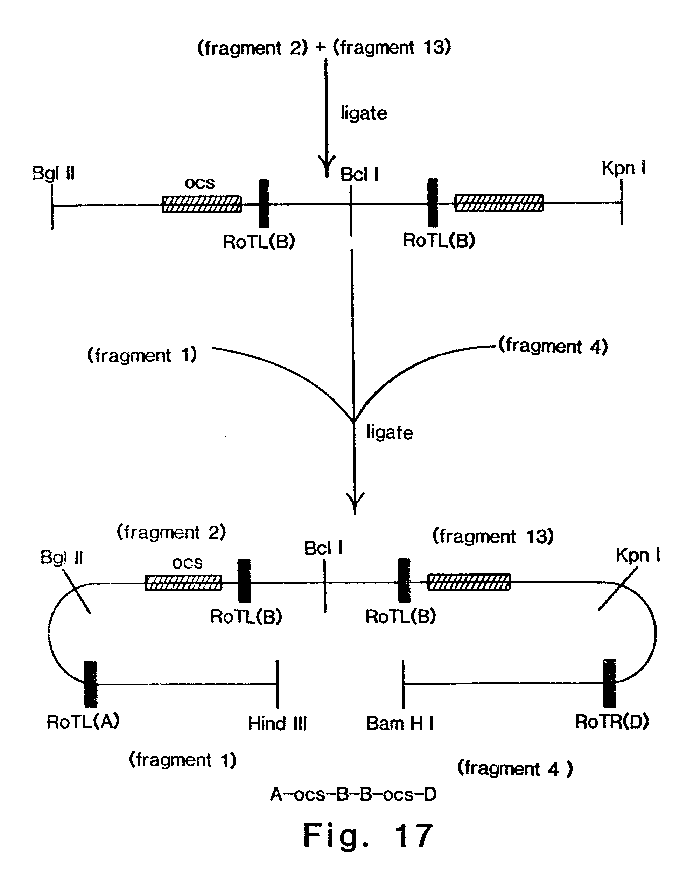

In the present example, fragments 2 and 13 are ligated together to give fragment 14 containing two octopine synthase genes and two repetitive RoTL(B) sequences in opposite orientations (Fig. 17). Following repurification of the ligated fragment, the correct orientation is checked by a restriction endonuclease map and the purified, ligated fragment 14 is then mixed with fragments 1 and 4 to yield fragment 15 (Fig. 17) containing all the required elements. Fragment 15 is inserted into the wide host range mutant pSUP106 following linearization of the vector with the restriction endonucleases HindIII and BamHI. The presence of two octopine synthase genes available for selection will increase the ability to select transformed cells or protoplasts from a mixture of transformed and normal cells or protoplasts when they are grown in the presence of a toxic anino acid such as canavanine or 2-amino-ethyl-cysteine.

### Example 8: Preparation of a Bacillus thuringiensis crystal protein gene

pES1 (H. E. Schnepf et al., Eur. Pat. Appl. 63,949, ATCC 31995) is cut with PstI, then mixed with and ligated to PstI-linearized mWB2344 (W. M. Barnes, et al. (1983) Nucleic Acids Res. 11:349-368). The resultant mixture is transformed into E. coli JM103, and tetracycline resistant transformants are selected. Double-stranded RFs (replicative form) are isolated from the transformants and characterized by restriction mapping. Two types of transformants are found: cells harboring mWB2344-ES1-A; and cells harboring mWB2344-ES1-S. These are M13 vectors which when in single-stranded viral form carry the ant sense and sense strands of the crystal protein gene of pES1.

The sequence of the crystal protein gene (see below: b) (H. C. Wong et al. (1983) J. Biol. Chem. 258:1960-1967), if changed at three base-pairs, will have a ClaI site (5'...AT*CGAT...3') immediately ahead of the ATG translational start site. The oligonucleotide (see below: a) 5'ATGGAGGTAATCGATGGATAACA3' is synthesized by standard methods, is hybridized to the single strand viral form of mWB2344-ES1-A, and is used to prime synthesis of a second strand of DNA by the Klenow fragment of E. coli DNA polymerase I. After ligation and selection of covalently closed circular DNA, the mixture is transformed into JM103. RF DNAs are isolated from the infected cells and characterized by restriction enzyme analysis. A clone descended from and containing the mutant sequence (a) is identified by the presence of a novel ClaI site which maps at the 5'-end of the crystal protein gene and is labeled mWB2344-ES1-A(Cla). The effect of the mutation can be seen by comparing the sequences of the oligonucleotide primer (a) with the 5' end of the crystal protein gene (b): 
a)          5'ATGGAGGTAAT*CG ATG GAT AAC A3'
b)  5'...AGAGATGGAGGTAAC TT/ATG/GAT/AAC/AAT/CC...3'
                                    Met Asp Asn Asn Pro...
Note that only three out of 23 base pairs have been changed (the underlined nucleotides of (a)), thereby assuring good hybridization properties.

The crystal protein gene is removed from mWB2344-ES1-A(Cla) by digestion with ClaI and XhoI. The XhoI sticky ends are converted to ClaI sticky ends by ligation to a linker, synthesized by standard methods, having a structure as follows: 
XhoI     ClaI
5'TCGAGCCCAT3'
   3'CGGGTACG5'
Excess linkers are trimmed off of the crystal protein gene-bearing fragment by digestion with ClaI.

### Example 9: Construction and modification of a promoter vehicle

pKS111, which is a pRK290 clone corresponding to the T-DNA clone p403 (Fig. 3) which encodes a gene covering 1.6kb (C. F. Fink (1982) M.S. thesis, University of Wisconsin-Madison), or p403 itself, is digested with ClaI and then religated. The ligation mix is transformed into E. coli K802 (W. B. Wood (1966) J. Mol. Biol. 16:118) and selected for tetracycline resistance. Piasmids are isolated by doing "minipreps" (plasmid preparations from small volume cell cultures) and restriction maps are obtained to prove the structure. The new vehicle, pKS-proI (see T. C. Hall et al., U.S. Patent Application Serial No. 485,614, incorporated herein by reference), can be linearized by ClaI.

The above manipulations are done with the following rationale: The T-DNA gene in pKS111 is shown below in summarized form as follows: 
ClaI 960 bp 250 bp ClaI 60 bp 50 bp
5'...TACACCAAAT*CG/ATG/GAC/ATG/..../TGA/.....AT*CGAT.....AAATAA....AAATAA...3'
promoter Met Asp Met.....stop polyadenylation signals
"1.6" kbp gene
By removing the ClaI fragment, the promoter region of the "1.6" gene is brought next to the 3'-downstream region of the gene. This 3' region includes polyadenylation signals. The resulting structure is summarized as follows:

pRK290
ClaI    60 bp              50 bp
5'...ATACACCAAAT*CGATAGT..........AAATAA..........AAATAAAA...3'    (pKSproI)
promoter                          polyadenylation signals

### Example 10: Insertion of the prol promoter into the A-ocs-B plasmid (Fig. 8)

pKS-proI is digested with EcoRI and mixed with and ligated to EcoRi/BamHI linkers, synthesized by standard procedures having the following structure: 
EcoRI    BamHI
5'AATTCCCCG3'
   3'GGGGCCTAG5'
The linkers are trimmed by digestion with BamHI. After the T-DNA promoter fragment is purified by gel electrophoresis, it is mixed with and ligated to BglII-linearized A-ocs-B (Example 2, Fig. 8). The mixture is transformed into E. coli GM33 and transformants resistant to chloramphenicol are selected. Plasmids isolated from such transformants are isolated and characterized by restriction enzyme analysis. A plasmid containing the promoter fragment is labeled pA-ocs-B-proI.

### Example 11: Insertion of the crystal protein gene into the vector

pA-ocs-B-proI is linearized with ClaI, and is then mixed with and ligated to the crystal protein gene-bearing fragment terminated by ClaI sticky ends constructed above. The resulting plasmids are transformed into GM33. Plasmlds isolated from transformants resistant to chloromphenicol are characterized by restriction analysis. A colony is chosen which contains a plasmid, pA-ocs-B-proI-ES1, having present a single copy of the crystal protein gene oriented in the same polarity as the proI promoter.

pA-ocs-B-proI-ES1 is transferred to an appropriate Agrobacterium host and used to transform tobacco cells as described in Example 12.

### Example 12: Selection of plant cells transformed by transfer of reconstructed T-DNA recombinant plasmids from Agrobacterium tumefaciens

The purpose of this example is to demonstrate a procedure to select transformed cells from mixtures of transformed and non-transformed cells. Generally, transformed cells are selected for their hormone autonomous growth. However, when Ti plasmids that have been mutated in tms, tmr or tml are used, then transformed cells are not autonomous and a selectable marker becomes desirable. Kanamycin or G418 resistance is a possibility, but it requires engineering a resistance gene. Another possibility is the use of octopine synthase as an enzyme to detoxify exogenously added toxic amino acid analogs, e.g., 2-aminoethyl-cysteine (2AEC). In this invention, it has been demonstrated that non-transformed tissues were killed by low levels of AEC (Fig. 5) whereas crown gall tissues expressing octopine synthase were not killed.

Therefore, the present example uses Ti-plasmids that are mutated in tms, tmr or tml but contain a non-mutated octopine synthase gene. Plants are first stem inoculated as previously described (K. A. Barton et al. (1983) Cell 32:1033-1043). After 10-12 days, fresh growth is removed and shaken in liquid culture until a number of cells have sloughed off. The culture is then passed through a filter and the small clumps of cells are collected. These clumps are plated on a filter paper placed on top of a feeder culture containing hormones.

Once the cells have started to grow, the entire filter is transferred to hormone media containing 2AEC. The transformed cells will be expressing octopine synthase that will detoxify the amino acid analog thus allowing them to grow whereas the untransformed cells will be killed.

Colonies that grow on 2AEC are picked and tested for octopine synthase. These cells can then be regenerated and can be shown to carry T-DNA containing the octopine synthase gene.

### Example 13: Tests for the relative toxicities of amino acid analogs using strains 15955/1 and 15955/01 from Nicotiana tabacum (tobacco) cv. "Xanthi"

Aqueous solutions of the amino acid analogs were adjusted to pH 5.6-5.8 and sterilized by filtration (0.45 micron Millipore filter). Serial dilutions of each analog were added to cytokinin- and auxin-free agar medium (Linsmaier, E. M. and F. Skoog (1965) Physioi. Plant 8:100-127) that had been autoclaved and allowed to cool to about 60°C. A number of other plant strains were used for initial screening of the analogs. Three 100mg pieces of 4-6 week old tissue were planted on analog-containing medium in 55 mm Petri dishes using tissue lines from Helianthus annuus (sunflower) cv. "Russian Mammoth" Kemp, J. D. (1982) In Kahl, G. and J. S. Schell (eds.) Academic Press, New York, London, Paris pp.461-474); E228 and Bo542, from N. tabacum cv. "Samsur (Sacristan, M. D. and G. Melchers (1977) Mol. Gen. Genet. 152:111-117), Braun's teratoma from N. tabacum cv. "Havana" (Braun, A. C. and H. N. Wood (1976) Proc. Nat. Acad. Sci. U.S.A. 73:496-500); A66 isolated from N. tabacum cv. "White Burley" (Firmin, J. L. and G. R. Fenwick (1978) Nature, London 276:842-844); and W-A6, W-B634, W-C58 and W-T37 isolated by J. Tournew, CNRA, Versailles, from tumors incited on N. tabacum cv. "Wisconsin 38" by octopine-type A. tumefaciens strains A6 or B634, or nopaline-type strains C58 or T37, respectively. The results with all of the above strains were essentially the same as those described for N. tabacum cv. "Xanthi" strains 15955/1 and 15955/01. For the toxicity studies with the 15955/1 and 15955/01 tumor lines, three 50mg pieces of 4-6 week old tissue were planted on medium in 90mm dishes. In all cases, duplicate or triplicate dishes were used for each tissue and each concentration of an analog. The tissues were weighed after 5-7 weeks of growth at 25°C in the dark. Growth was expressed as a percentage of the fresh weight increase of the same tissue on medium that did not contain any analog or amino acid. The experiments were repeated after initial results delimited the range of ccncentrations to use for a given analog. Experiments with the 15955/1 and 15955/01 lines were repeated at least twice with the appropriate concentration ranges.

### Example 14: Method of assaying for opine synthases

Octopine synthase was assayed using some modifications of previous methods (Birnberg, P. et al. (1977) Phytochemistry 16:647-650; Goldmann, A. (1977) Plant Sci. Lett. 10:49-58; Hack, E. and J. D. Kemp (1977) Biochem. Biophys. Res. Comms. 78:785-791; Lejeune, B. (1967) C. R. Acad. Sci. Ser. D. 265:1753-1755; Otten, L. A. B. M. and R. A. Schilperoort (1978) Biochim. Biophys. Acta. 527:497-500). For each mg of tissue in a 1.5ml Eppendorf tube, 3µl of a buffered (0.15M potassium phosphate, pH 6.9) reaction mixture was added. This reaction mixture contained 20 mM L-arginine, 50 mM pyruvate and 13.5 mM NADH. The tissue was carefully macerated in the reaction mixture with a glass rod and incubated at 25°C for one hour. The samples were then clarified by centrifugation and 2µl of supernate was spotted on Whatman 3MM paper. Up to 30µl of sample was used to verify a negative assay result. The Whatman 3MM paper with the samples, as well as 1-10µg of octopine (or other opine) as an authentic standard and Orange G as a migration marker, was carefully wetted with electrophoresis buffer (formic acid/acetic acid/water; 3/6/91, v/v/v) and electrophoresed at 50-75 volts/cm (Gibson High Voltage Electrophoresis Apparatus, Model D) for 10-20 minutes. The electrophoretograms were dried in a current of hot air and stained for octopine (or other opines) with either a phenanthrene quinone reagent (Yamada, S. and H. A. Itano (1966) Biochim. Biophys. Acta. 130:538-540) or with Sakaguchi reagent (Easley, C. W. (1965) Biochim. Biophys. Acta. 107:386-388). The Sakaguchi reagent is several-fold less sensitive but was more specific for guanidinyl compounds. When the above conditions were used, it was also shown that tissue extraction buffers (Hack, E. and J. D. Kemp (1977) supra; Otten, L. A. B. M. and R. A. Schilperoort (1978) supra) did not improve assay results and, in fact, retarded electrophoretic migration when larger sample volumes were spotted. Electrophoretic mobilities were measured from the origin (0) relative to Orange G (1.0).

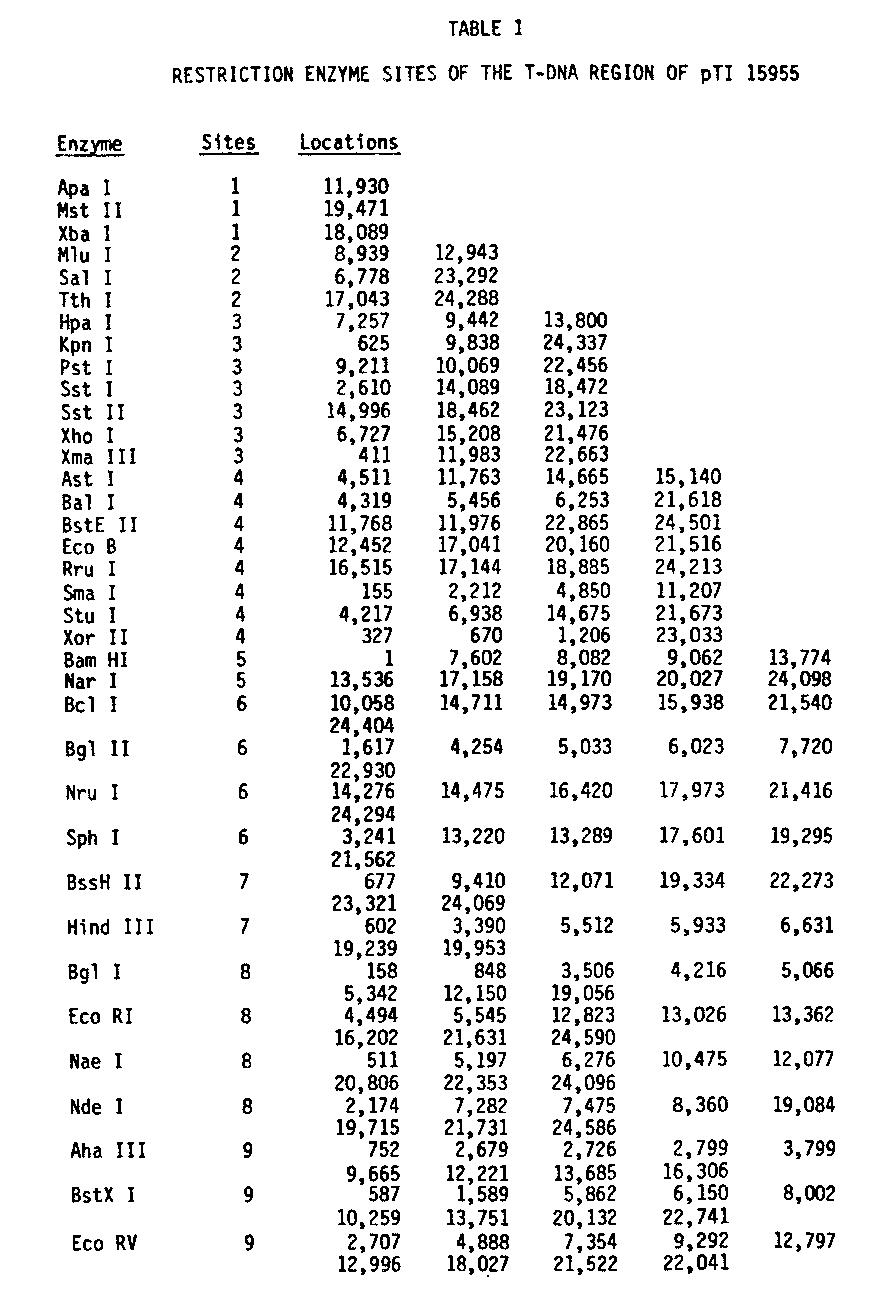

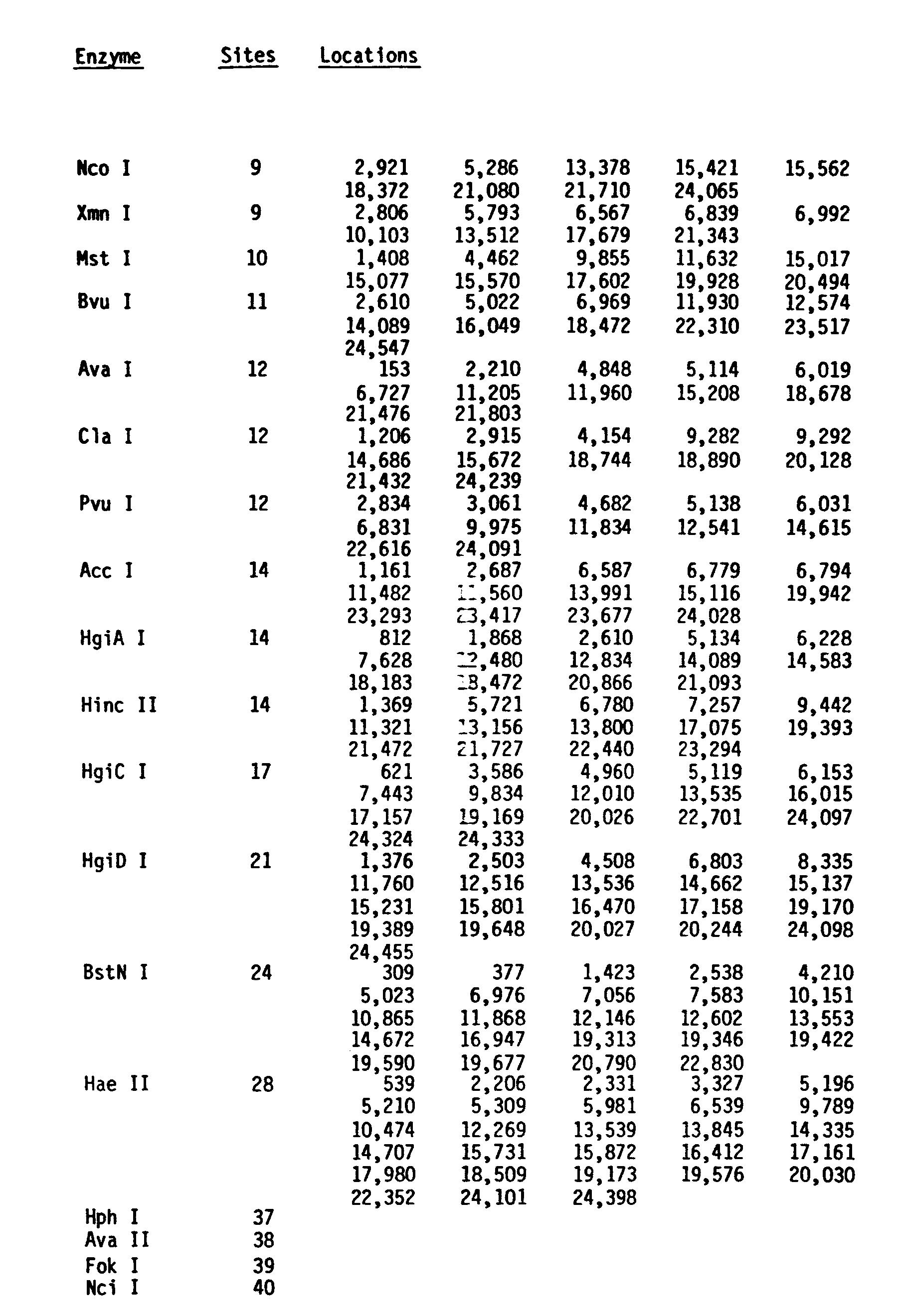

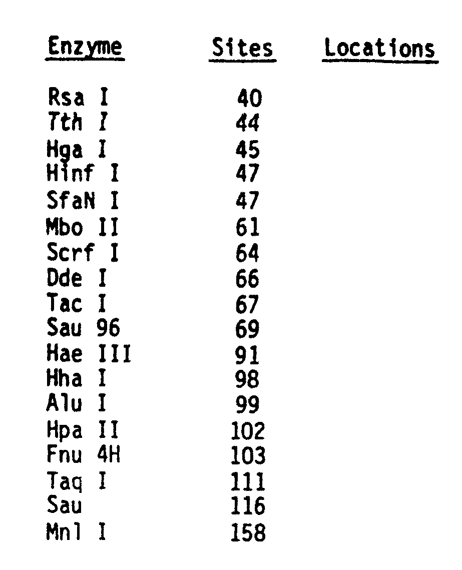

**TABLE 2**

| A list of the tissues used for testing ability to grow in the presence of various toxic amino acid analogs. | | | | |
|---|---|---|---|---|
| Strain No. | Cultivar | Transformed by: | Mannopine | Agropine |
| 159 No. 1 | Nicotiana tabacum | Agrobacterium tumefaciens | - | - |
| | cv. Xanthi | strain 15955 | | |
| 1590-1 | do | do | + | + |
| W-C58 | N. tabacum | A. tumefaciens | - | - |
| | cv. Wisc. 38 | strain C58 | | |
| W-B634 | N. tabacum | A. tumefaciens | + | + |
| | cv. Wisc. 38 | strain B634 | | |

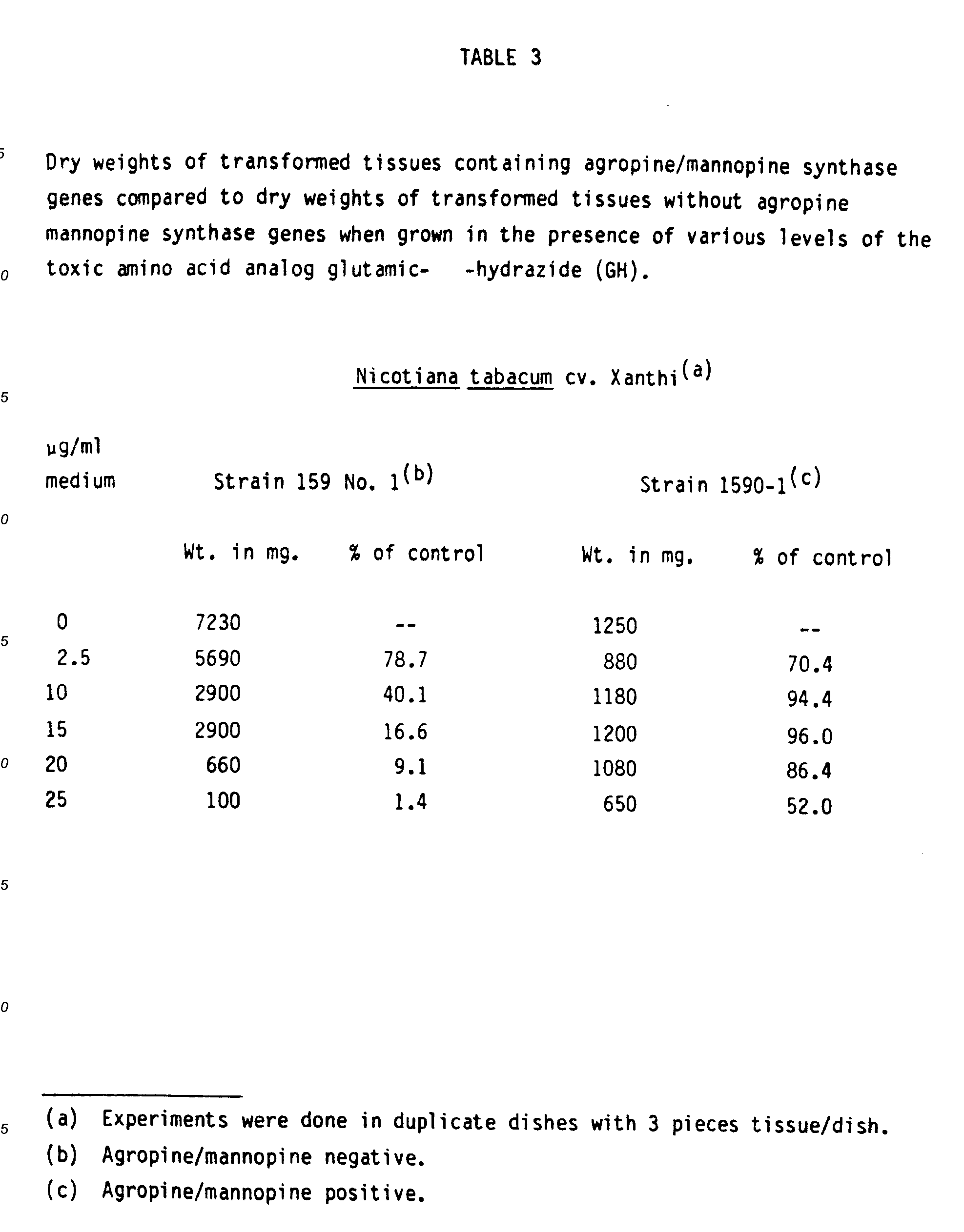

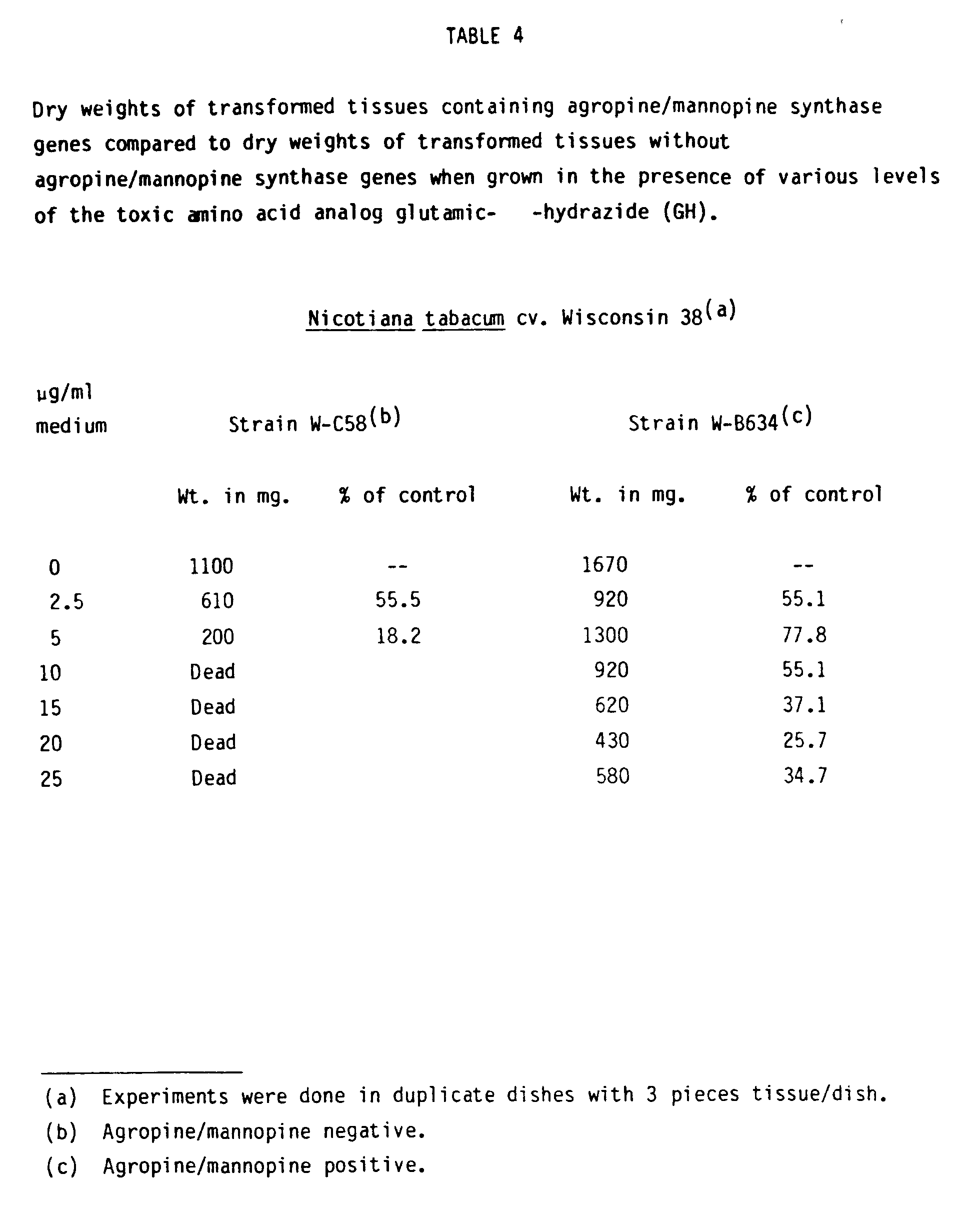

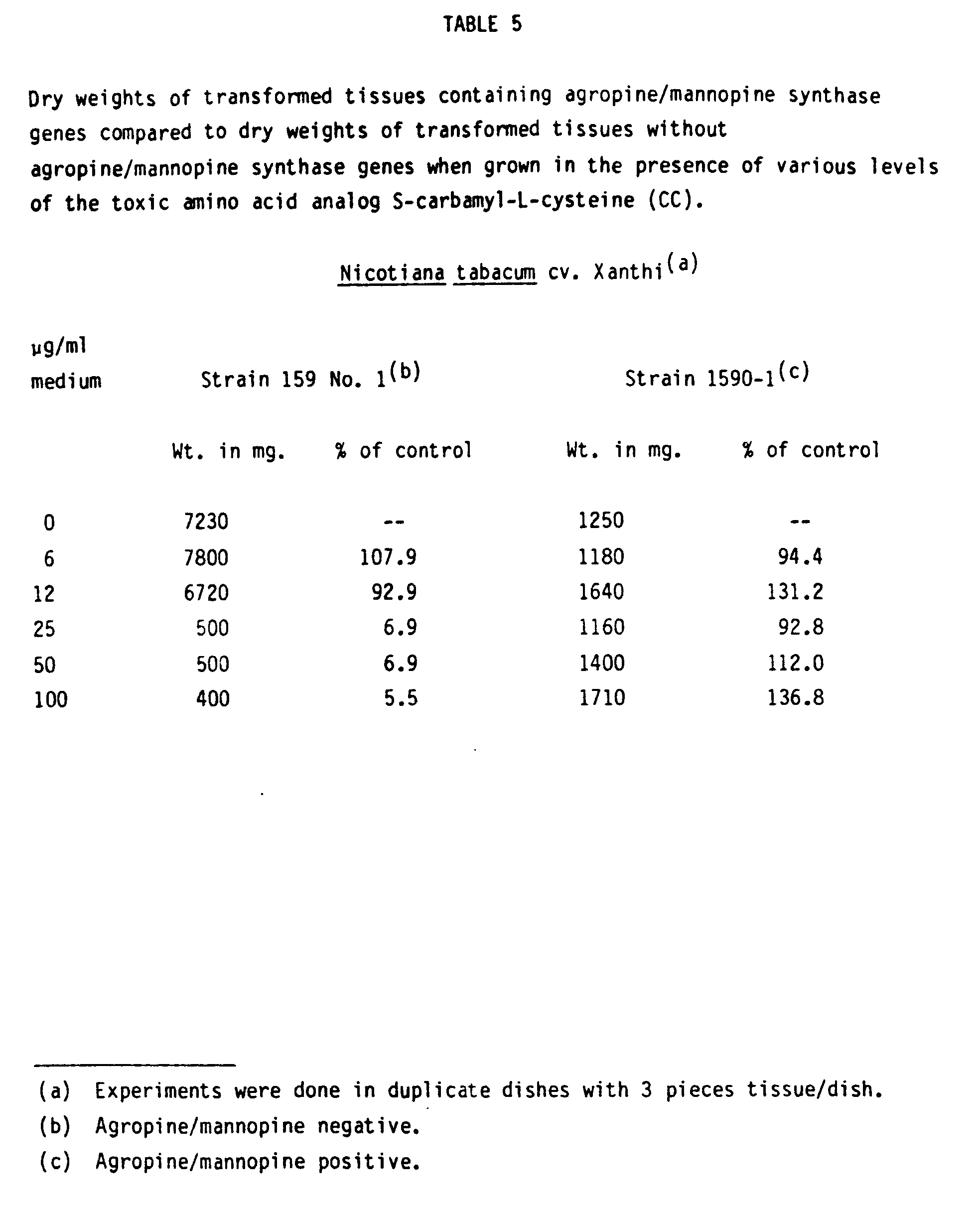

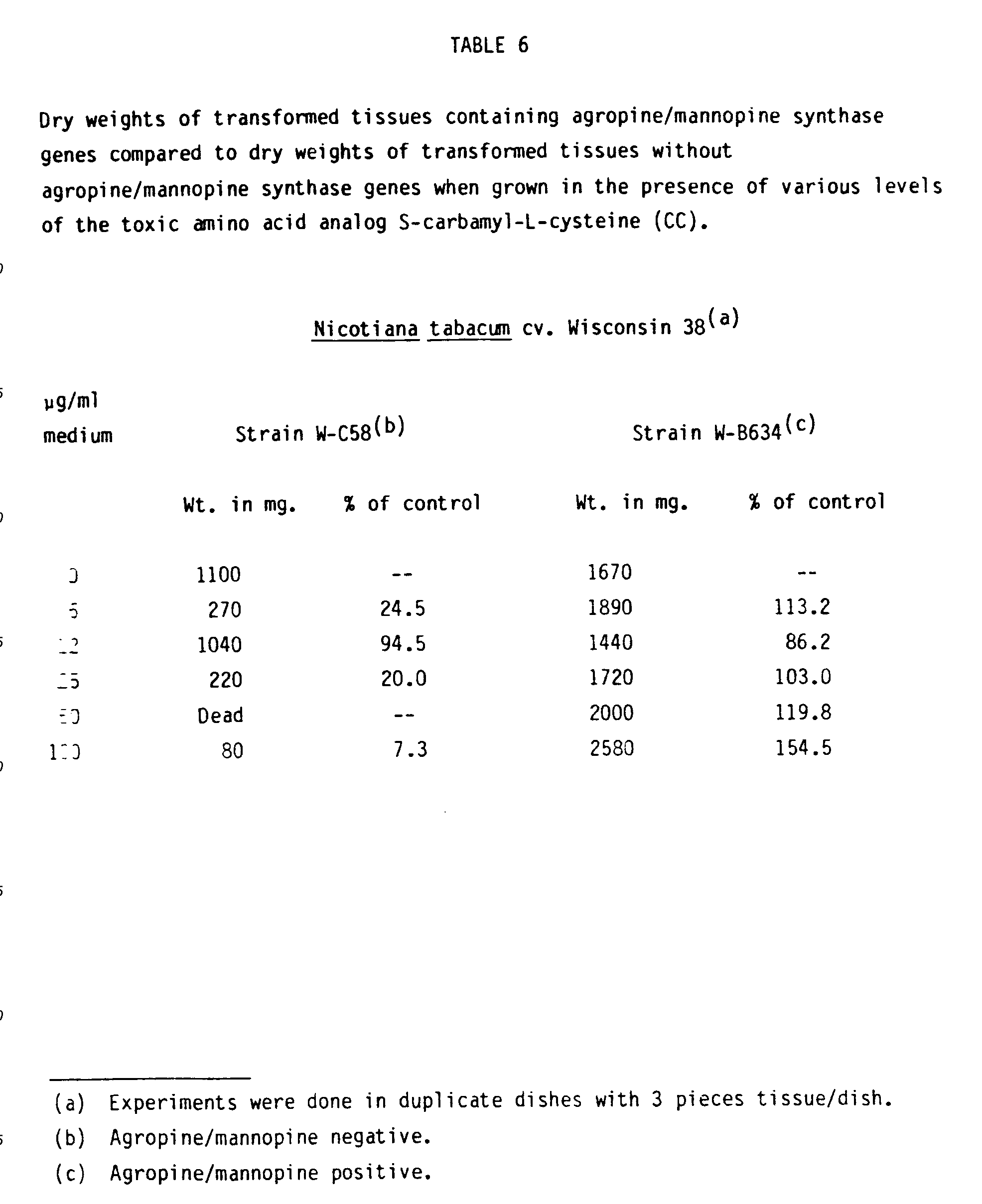

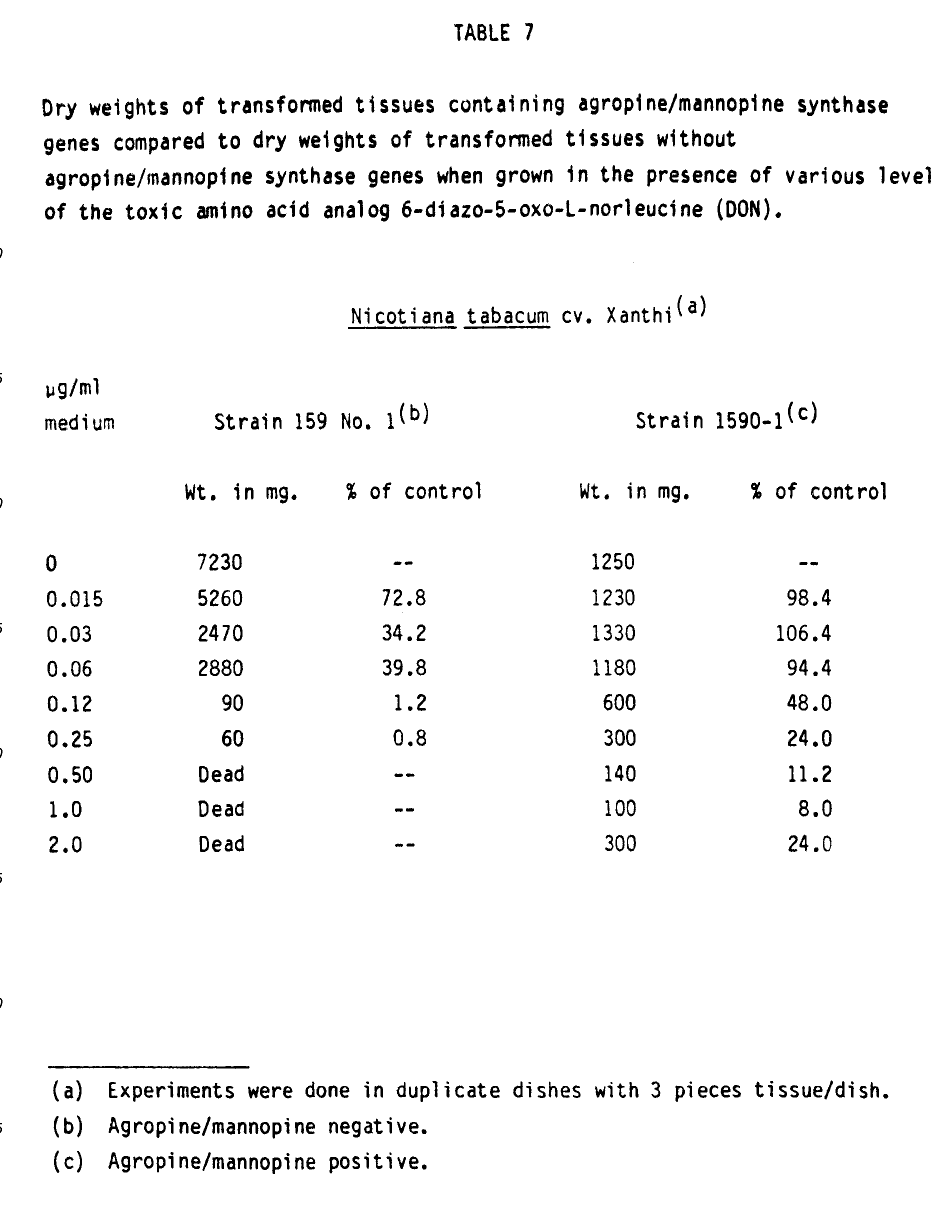

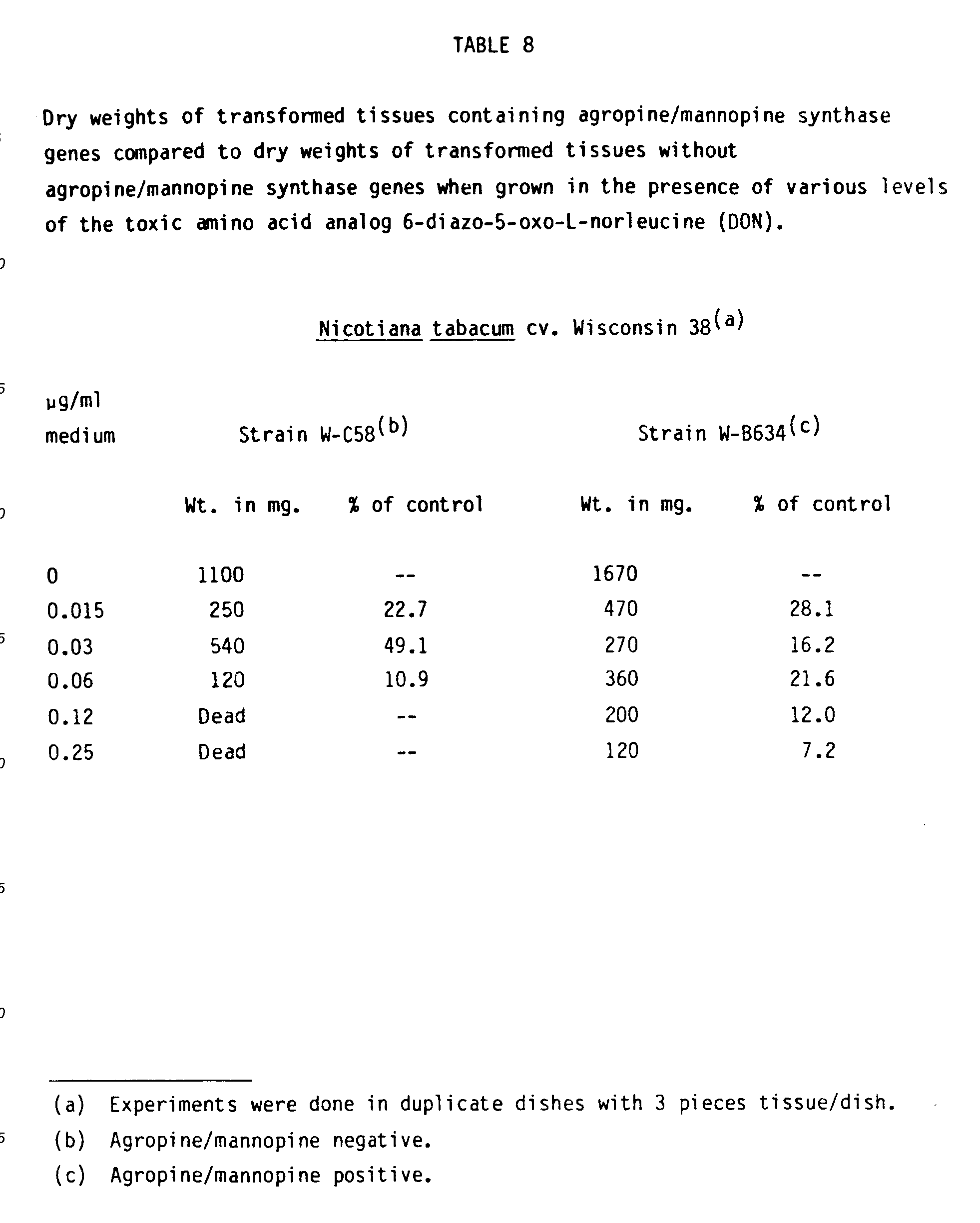

## Claims

1. A vector plasmid for the transfer of foreign DNA into the genome of recipient plant cells comprising a heterologous DNA sequence which is expressible in a plant, which is flanked on each side by a T-DNA repetitive sequence selected from the group consisting of RoTL(A), RoTL(B), RoTR(C) and RoTR(D) having the following nucleotide sequences:

RoTL(A): G G C A G G A T A T A T T C A A T T G T A A A T;

RoTL(B): G G C A G G A T A T A T A C C G T T G T A A T T;

RoTR(C): G G C A G G A T A T A T C G A G G T G T A A A A;

RoTR(D): G G C A G G A T A T A T G C G G T T G T A A T T.

2. The vector plasmid of claim 1 wherein at least one of said flanking T-DNA repetitive sequences is RoTL(B).

3. The vector plasmid of claim 1 wherein at least one of said flanking T-DNA repetitive sequences is RoTR(D).

4. A vector plasmid of any of claims 1 to 3 wherein said plasmid lacks tumor-forming genes and further lacks antibiotic resistance genes in the T-DNA region; and which comprises at least one opine synthase gene that is expressible in a plant.

5. A plasmid according to claim 4 wherein said heterologous DNA is expressed under the control of a heterologous promoter.

6. A plasmid according to claim 4 wherein said heterologous DNA is expressed under the control of an opine synthase promoter.

7. A plasmid according to claim 4 wherein at least one of said opine synthase gene or genes are selected independently from octopine synthase genes and agropine/mannopine synthase genes.

8. A plant cell transformed by a plasmid according to any preceding claim.

9. A plant or plant tissue comprising plant cells according to claim 8.

10. A method of selecting non-tumorous plant cells transformed with a DNA sequence comprising a plant expressible opine synthase gene and a plant expressible heterologous gene, said sequence being flanked on each side by a T-DNA repetitive sequence selected from the group consisting of RoTL(A), RoTL(B), RoTR(C) and RoTR(D) having the nucleotide sequences as defined in claim 1 from a mixture containing said transformed plant cells and untransformed plant cells comprising:
(a) plating said mixture on a suitable growth medium containing an amino acid analog toxic to normal cells and metabolized by a plant cell expressing the opine synthase encoded by said gene;
(b) growing said mixture on said growth medium for a selected period of time to provide colonies of plant cells; and
(c) selecting from said colonies those colonies exhibiting greater growth rates.

11. The method of claim 10 wherein said transformed plant cells comprise a DNA segment comprising:
(a) at least two T-DNA repetitive sequences at least one of which is RoTL(B);
(b) at least one opine synthase gene that is expressible in a plant; and
(c) at least one heterologous gene that is expressible in a plant, wherein said DNA segment lacks tumor-forming and antibiotic resistance genes.

12. The method of claim 10 wherein said transformed plant cells comprise a DNA segment comprising:
(a) at least two T-DNA repetitive sequences at least one of which is RoTR(D);
(b) at least one opine synthase gene that is expressible in a plant; and
(c) at least one heterologous gene that is expressible in a plant, wherein said DNA segment lacks tumor-forming and antibiotic resistance genes.

13. The method of any of claims 10 to 12 wherein said amino acid analog is canavanine.

14. The method of any of claims 10 to 12 wherein said amino acid analog is 2-amino-ethyl-cysteine.

15. The method of any of claims 10 to 12 wherein said amino acid analog is glutamic-gamma-hydrazide.

16. The method of any of claims 10 to 12 wherein said amino acid analog is S-carbamyl-L-cysteine.

17. The method of any of claims 10 to 12 wherein said amino acid analog is 6-diazo-5-oxo-1-norleucine.

18. A DNA fragment comprising the sequence of RoTR(D) as follows:
G G C A G G A T A T A T G C G G T T G T A A T T.

19. A DNA fragment comprising the sequence of RoTL(B) as follows:
G G C A G G A T A T A T A C C G T T G T A A T T.

## Revendications

1. Un plasmide vecteur pour le transfert d'ADN étranger dans le génome de cellules d'une plante réceptrice comprenant une séquence d'ADN hétérologue qui est exprimable dans une plante, qui est flanqué de chaque côté par une séquence répétitive de T-ADN choisie dans le groupe constitué par RoTL(A), RotL(B), RoTR(C) et RoTR(D) ayant les séquences de nucléotides suivantes:

RoTL(A): G G C A G G A T A T A T T C A A T T G T A A A T;

RoTL(B): G G C A G G A T A T A T A C C G T T G T A A T T;

RoTR(C): G G C A G G A T A T A T C G A G G T G T A A A A;

RoTR(D): G G C A G G A T A T A T G C G G T T G T A A T T.

2. Le plasmide vecteur de la revendication 1, dans lequel au moins une desdites séquences répétitives de T-ADN flanquantes est RoTL(B).

3. Le plasmide vecteur de la revendication 1, dans lequel au moins une desdites séquences répétitives de T-ADN flanquantes est RoTR(D).

4. Un plasmide vecteur selon l'une quelconque des revendications 1 à 3 dans lequel le plasmide ne comporte pas de gênes formateurs de tumeurs et également de gênes de résistances aux antibiotiques dans la région de T-ADN et qui comprend au moins un gêne de synthase opine qui est exprimable dans une plante.

5. Un plasmide selon la revendication 4 dans lequel ledit ADN hétérologue est exprimé sous le contrôle d'un promoteur hétérologue.

6. Un plasmide selon la revendication 4 dans lequel ledit ADN hétérologue est exprimé sous le contrôle d'un promoteur de synthase opine.

7. Un plasmide selon la revendication 4, dans lequel au moins un dudit gêne ou desdits gênes de synthase opine sont choisis indépendamment des gênes de synthase octopine et des gênes de synthase agropine/mannopine.

8. Une cellule de plante transformée par un plasmide selon l'une quelconque des revendications précédentes.

9. Une plante ou un tissu de plante comprenant des cellules de plante selon la revendication 8.

10. Une méthode pour sélectionner des cellules de plante sans tumeurs transformées au moyen d'une séquence d'ADN comprenant un gêne de synthase opine exprimable par la plante et un gêne hétérologue exprimable par la plante, ladite séquence étant flanquée de chaque côté par une séquence répétitive de T-ADN choisie dans le groupe consistant en RoTL(A), RoTL(B), RoTR(C) et RoTR(D) ayant les séquences de nucléotides telles que définies dans la revendication 1 à partir d'un mélange contenant lesdites cellules de plante transformées et les cellules de plante non transformées comprenant:
(a) l'application en plaque dudit mélange sur un milieu de culture approprié contenant un analogue d'acide aminé toxique vis-à-vis des cellules normales et métabolisé par une cellule de plante exprimant la synthase opine codée par ledit gêne;
(b) la culture dudit mélange sur le milieu de culture pendant une période de temps choisie pour fournir des colonies de cellules de plante; et
(c) la sélection, à partir desdites colonies, de celles qui exhibent les vitesses de croissance les plus grandes.

11. La méthode de la revendication 10 dans laquelle les cellules de plante transformées comprennent un segment d'ADN comprenant:
(a) au moins deux séquences répétitives de T-ADN dont au moins une est RoTL(B);
(b) au moins un gêne de synthase opine exprimable dans une plante; et
(c) au moins un gêne hétérologue qui est exprimable dans une plante, dans lequel lesdits segments d'ADN ne comportent pas de gênes formateurs de tumeurs et de résistance aux antibiotiques.

12. La méthode selon la revendication 10 dans laquelle lesdites cellules de plante transformées comprennent un segment d'ADN comprenant:
(a) au moins deux séquences répétitives de T-ADN dont au moins une est RoTR(D);
(b) au moins un gêne de synthase opine exprimable dans une plante; et
(c) au moins un gêne hétérologue qui est exprimable dans une plante, dans lequel ledit segment d'ADN ne comporte pas de gênes formateurs de tumeurs et de résistance aux antibiotiques.

13. La méthode selon l'une quelconque des revendications 10 à 12, dans laquelle ledit analogue d'acide aminé est la canavanine.

14. La méthode selon l'une quelconque des revendications 10 à 12, dans laquelle ledit analogue acide aminé est la 2-amino-éthyl-cystéine.

15. La méthode selon l'une quelconque des revendications 10 à 12, dans laquelle ledit analogue acide aminé est le gamma-hydrazide glutamique.

16. La méthode selon l'une quelconque des revendications 10 à 12, dans laquelle ledit analogue acide aminé est la S-carbamyl-L-cystéine.

17. La méthode selon l'une quelconque des revendications 10 à 12, dans laquelle ledit analogue acide aminé est la 6-diazo-5-oxo-1-norleucine.

18. Un fragment d'ADN comprenant la séquence de RoTR(D) comme suit:
G G C A G G A T A T A T G C G G T T G T A A T T.

19. Un fragment d'ADN comprenant la séquence de RoTL(B) comme suit:
G G C A G G A T A T A T A C C G T T G T A A T T.

## Patentansprüche

1. Vektorplasmid für den Transfer von Fremd-DNA in das Genom von pflanzlichen Aufnahmezellen, umfassend eine heterologe, pflanzenexprimierbare DNA-Sequenz, die beidseits von einer sich wiederholenden T-DNA-Sequenz flankiert ist, die aus der Gruppe RoTL(A), RoTL(B), RoTR(C) und RoTR(D) ausgewählt ist, wobei diese die folgenden Nucleotidsequenzen haben:

RoTL(A): G G C A G G A T A T A T T C A A T T G T A A A T;

RoTL(B): G G C A G G A T A T A T A C C G T T G T A A T T;

RoTR(C): G G C A G G A T A T A T C G A G G T G T A A A A;

RoTR(D): G G C A G G A T A T A T G C G G T T G T A A T T.

2. Vektorplasmid nach Anspruch 1, bei welchem zumindest eine der flankierenden, sich wiederholenden T-DNA-sequenzen RoTL(B) ist.

3. Vektorplasmid nach Anspruch 1, bei welchem zumindest eine der flankierenden, sich wiederholenden T-DNA-Sequenzen RoTR(D) ist.

4. Vektorplasmid nach einem der Ansprüche 1 bis 3, welchem tumorbildende Gene und außerdem antibiotische Resistenzegene in der T-DNA-Region fehlen; und welches zumindest ein pflanzenexprimierbares Opinsynthase-Gen umfaßt.

5. Plasmid nach Anspruch 4, in welchem die heterologe DNA unter der Steuerung eines heterologen Promotors exprimiert wird.

6. Plasmid nach Anspruch 4, in welchem die heterologe DNA unter der Steuerung eines Opinsynthase-Promotors exprimiert wird.

7. Plasmid nach Anspruch 4, in welchem zumindest ein Opinsynthase-Gen oder eines der Opinsynthase-Gene unabhängig voneinander ausgewählt ist aus den Octopinsynthase-Genen und den Agropin-/Mannopinsynthase-Genen.

8. Pflanzenzelle, die mit einem Plasmid nach einem der vorhergehenden Ansprüche transformiert ist.

9. Pflanze oder Pflanzengewebe, die bzw. das eine Pflanzenzelle nach Anspruch 8 enthält.

10. Verfahren zur Selektierung nicht tumoröser Pflanzenzellen, die mit einer DNA-Sequenz transformiert sind, die ein pflanzenexprimierbares Opinsynthase-Gen und ein pflanzenexprimierbares heterologes Gen umfaßt, wobei diese Sequenz beidseits von einer sich wiederholenden T-DNA-Sequenz flankiert ist, die aus der Gruppe RoTL(A), RoTL(B), RoTR(C) und RoTR(D) mit den in Anspruch 1 definierten Nucleotidsequenzen ausgewählt ist, aus einer Mischung, die diese transformierten Pflanzenzellen und nicht transformierte Pflanzenzellen enthält, wobei:
(a) diese Mischung auf ein geeignetes Wachstumsmedium aufgestrichen wird, das ein für normale Zellen toxisches und von eine Pflanzenzelle, die die von dem genannten Gen codierte Opinsynthase exprimiert, metabolisiertes Aminosäureanalogon enthält;
(b) Züchtung dieser Mischung auf dem Wachstumsmedium während eines bestimmten Zeitraums, um Kolonien der Pflanzenzellen zu bilden; und
(c) Selektion jener Kolonien, die größere Wachstumsraten zeigen, aus diesen Kolonien.

11. Verfahren nach Anspruch 10, bei welchem die genannten transformierten Pflanzenzellen ein DNA-Segment umfassen, das enthält:
(a) zumindest zwei sich wiederholende T-DNA-Sequenzen, von denen zumindest eine RoTL(B) ist;
(b) zumindest ein Opinsynthase-Gen, das in einer Pflanze exprimierbar ist; und
(c) zumindest ein heterologes, pflanzenexprimierbares Gen, wobei diesem DNA-Segment tumorbildende und antibiotische Resistenzgene fehlen.

12. Verfahren nach Anspruch 10, bei welchem diese transformierten Pflanzenzellen ein DNA-Segment aufweisen, das enthält:
(a) zumindest zwei sich wiederholende T-DNA-Sequenzen, von denen zumindest eine RoTR(D) ist;
(b) zumindest ein Opinsynthase-Gen, das in einer Pflanze exprimierbar ist; und
(c) zumindest ein heterologes, pflanzenexprimierbares Gen, wobei diesem DNA-Segment tumorbildende und antibiotische Resistenzgene fehlen.

13. Verfahren nach einem der Ansprüche 10 bis 12, bei welchem dieses Aminosäureanalogon Canavanin ist.

14. Verfahren nach einem der Ansprüche 10 bis 12, bei welchem dieses Aminosäureanalogon 2-Aminoethylcystein ist.

15. Verfahren nach einem der Ansprüche 10 bis 12, bei welchem dieses Aminosäureanalogon Glutaminsäure-gamma-hydrazid ist.

16. Verfahren nach einem der Ansprüche 10 bis 12, bei welchem dieses Aminosäureanalogon S-Carbamyl-L-cystein ist.

17. Verfahren nach einem der Ansprüche 10 bis 12, bei welchem dieses Aminosäureanalogon 6-Diazo-5-oxo-1-norleucin ist.

18. DNA-Fragment, enthaltend die Sequenz von RoTR(D) wie folgt:
G G C A G G A T A T A T G C G G T T G T A A T T.

19. DNA-Fragment, enthaltend die Sequenz von RoTL(B) wie folgt:
G G C A G G A T A T A T A C C G T T G T A A T T.
